# EUROPÄISCHE PATENTSCHRIFT

(11) **EP 0 407 900 B1**
(45) Veröffentlichungstag und Bekanntmachung des Hinweises auf die Patenterteilung: **05.04.1995**
(21) Anmeldenummer: 90112904.9
(22) Anmeldetag: 06.07.1990
(51) Int. Cl.: B01D 71/34, B01D 67/00

(54) **Flach- oder Kapillarmembran auf der Basis eines homogenen Gemisches aus Polyvinylidenfluorid und eines zweiten, durch chemische Umsetzung hydrophilierbaren Polymeren**
Flat or capillary membrane manufactured from a mixture of polyvinylidene fluoride and a second by chemical reaction hydrophilable polymer
Membrane plate ou capillaire fabriquée d'un mélange homogène de fluorure de polyvinylidène et d'un autre polymère hydrophilable par réaction chimique

(30) Priorität: 13.07.1989 DE 3923128
(43) Veröffentlichungstag der Anmeldung: 16.01.1991
(73) Patentinhaber: Akzo Nobel N.V., 6824 BM Arnhem (NL)
(72) Erfinder: Müller, Heinz-Joachim, Dr., D-6550 Bad Kreuznach (DE); Sluma, Heinz-Dieter, Dr., D 8754 Grossostheim (DE); Eberhard, Günter, Dr., D-8765 Erlenbach (DE); Spindler, Ernst, Dr., D-4322 Sprockhövel 2 (DE); Krauss, Lothar, D-8765 Erlenbach (DE); Völker, Helmut, D-8763 Klingenberg (DE)
(74) Vertreter: Fett, Günter

(56) Entgegenhaltungen:
- EP-A- 0 186 758
- EP-A- 0 245 000
- DE-A- 2 735 887
- PATENT ABSTRACTS OF JAPAN Band 3, Nr. 32 (C-40), 17.März 1979; & JP - A - 548669 (SUMITOMO DENKI KOGYO K.K.) 23.01.1979

## Beschreibung

Die Erfindung bezieht sich auf eine Flach- oder Kapillarmembran auf der Basis eines homogenen Gemisches aus Polyvinylidenfluorid (PVDF) und eines zweiten, durch chemische Umsetzung hydrophilierbaren Polymeren und auf Verfahren zu ihrer Herstellung und weiteren chemischen Modifizierung.

Polyvinylidenfluoridmembranen, die insbesondere eine ausgezeichnete Wärmebeständigkeit und Beständigkeit gegen Chemikalien aufweisen, sind bekanntlich hydrophob und lassen sich für die Trennung wäßriger Lösungen schwierig anwenden. Es sind bereits im Stand der Technik zahlreiche Versuche unternommen worden, durch Modifikationen unterschiedlichster Art solche Membranen zu hydrophilieren.

So wird in der DE-A-27 35 887 ein Verfahren beschrieben, bei dem man die Poren eines porösen Fluorkohlenwasserstoffpolymeren mit mindestens einem wasserlöslichen Polymeren einschließlich Polyvinylalkohol imprägniert und den Polyvinylalkohol durch Wärmebehandlung oder ionisierende Strahlung in Wasser unlöslich macht.

Imprägnierungsverfahren besitzen jedoch den Nachteil, daß durch die Beschichtung der Membranporen die Membranstruktur zum Teil verstopft werden kann, wodurch die Flußwerte der Membranen ungünstig beeinflußt werden. Weiterhin ist eine Beschichtung von Polyvinylidenfluorid mit einem hydrophilen Polymer, zumal bei der Konstellation der Unverträglichkeit von Substrat und Beschichtung, nicht sehr beständig. Sie kann daher durch bestimmte Medien, insbesondere Schwefelsäure oder Hypochloritlösungen, die bei der Reinigung von Membranen bzw. in der Halbleiterindustrie benötigt werden, zerstört werden, woraus Nachteile resultieren, wie verstärkte Abgabe von Fremdsubstanzen bzw. Partikeln, und die Hydrophilie der Membranen unwiederbringlich verloren geht.

Die Verträglichkeit von PVDF mit hydrophilen Polymeren ist leider sehr begrenzt. So lassen sich zwar aus PVDF und einigen wenigen hydrophilen Polymeren, wie z.B. Polyvinylpyrrolidon, bei einem genügend hohen Gewichtsanteil der hydrophilen Komponente hydrophile Membranen herstellen. Sie besitzen aber eine sehr niedrige mechanische Festigkeit, und oft wird das hydrophile Polymer bei Anwendungsbedingungen aus der Membran extrahiert.

Auch durch Pfropfpolymerisation können PVDF-Membranen hydrophiliert werden. So wird in der EP-A-0 245 000 ein Verfahren beschrieben, bei dem PVDF-Membranen zunächst mit Alkalilauge behandelt werden, um durch Abspaltung von Fluorwasserstoff auf der Oberfläche der PVDF-Membran reaktive Stellen zu erzeugen. Auf diese werden dann unter Verwendung eines Polymerisationsinitiators polymerisierbare hydrophile Vinylmonomere, wie Acrylsäure, Methacrylsäure und Itaconsäure, aufgepfropft. Neben der Gefahr der Schädigung der Membran durch die Alkalilauge und einer Verstopfung der Poren durch das auspolymerisierte hydrophile Vinylmonomer ist bei dieser Verfahrensweise auch nachteilig, daß die Membran nach dem Pfropfen noch toxische Acrylmonomere und -oligomere enthält, die nur mit hohem Aufwand vollständig aus der Membran extrahiert werden können.

Zur Vermeidung der geschilderten Nachteile ist ein weiterer Weg bekannt geworden, wonach aus einem homogenen Gemisch aus PVDF und einem zweiten, durch chemische Umsetzung hydrophilierbaren Polymeren, das also mit PVDF in dem angewendeten Gewichtsbereich compatibel sein muß, eine Membran hergestellt und anschließend das zweite Polymer durch chemische Reaktion in ein hydrophiles umgewandelt wird. So wird in der EP-A-0 012 557 eine Membran aus einem homogenen Gemisch aus PVDF und Polyvinylacetat hergestellt und letzteres anschließend hydrolysiert, woraus eine hydrophile Membran resultiert, die aufgrund des entstandenen Polyvinylalkohols Hydroxylgruppen enthält. In dieser Patentanmeldung wird jedoch hervorgehoben, daß die Membranen mindestens 35 Gewichtsprozent Polyvinylacetat enthalten müssen, wenn sie nach erfolgter Hydrolyse einen ausreichend hydrophilen Charakter aufweisen sollen. Bevorzugt wird daher ein Polyvinylalkoholgehalt von 43 bis 67 Gewichtsprozent in der hydrophilen Membran, was einem ursprünglich vorhandenen Gewichtsanteil an Polyvinylacetat von 60 - 80 Gewichtsprozent entspricht. Bei derartig hohen Gewichtsanteilen Polyvinylalkohol bzw. Polyvinylacetat werden aber die eingangs beschriebenen günstigen Polymereigenschaften von PVDF naturgemäß erheblich verschlechtert.

Die vorliegende Erfindung stellt sich die Aufgabe, eine Flach- oder Kapillarmembran auf der Basis eines homogenen Gemisches aus PVDF und eines zweiten, durch chemische Umsetzung hydrophilierbaren Polymeren bereitzustellen, die sich durch einen erheblich niedrigeren Gewichtsanteil an dem zweiten Polymeren auszeichnet, der gewährleisten soll, daß einerseits die hervorragenden chemischen und physikalischen Gesamteigenschaften des PVDF praktisch erhalten bleiben und andererseits nach dessen chemischer Umsetzung dennoch ausreichende hydrophile Eigenschaften erhalten werden.

Überraschend wurde gefunden, daß als zweites Polymer schon der sehr geringe Gewichtsanteil von mindestens 2 Gewichtsprozent aus einem im wesentlichen aus Polyacrylsäuremethyl-und/oder -ethylester gebildeten Polymeren zur Lösung der gestellten Aufgabe ausreichend ist. Unter dem Begriff "ein im wesentlichen aus Polyacrylsäuremethyl- und/oder -ethylester gebildetes Polymer" werden insbesondere der reine Polyacrylsäuremethylester, der entsprechende Polyacrylsäureethylester, Gemische dieser zwei Polyacrylsäureester und sämtliche Mischpolymerisate aus den zwei Monomeren Acrylsäuremethylester und Acrylsäureethylester verstanden. Ferner werden hierin Mischpolymerisate aus den zuletzt genannten zwei Stoffen eingeschlossen, die dadurch entstehen, daß man die Acrylsäuremethylester- und/oder Acrylsäureethylester-Monomeren, die eine hervorragende Fähigkeit zur Mischpolymerisation besitzen, mit geringen Mengen mit an sich bekannten üblichen anderen Monomeren mischpolymerisiert. Hierfür geeignete Monomere, die im allgemeinen nur in Mengen von bis zu 10 Gewichtsprozent verwendet werden können, sind z.B. Acrylsäureamid, Acrylnitril, Maleinsäureester, Vinylacetat, Vinylpropionat, Methacrylate, Styrol und Butadien. Überraschend ist in diesem Zusammenhang auch, daß außer den zwei genannten Grundarten von Acrylsäureesterpolymeren die Polymeren aus Methacrylsäuremethylester und/oder -ethylester oder Ester der Acrylsäure oder Methacrylsäure mit Alkoholen, die mehr als zwei Kohlenstoffatome aufweisen, nicht geeignet sind, um die erfindungsgemäßen Membranen herzustellen. Derartige Monomeren können allenfalls, wie oben geschildert, als geringfügige Beimischung zu den erfindungsgemäß verwendeten Monomeren über die Bildung eines Copolymeren verwendet werden.

Die vorliegende Erfindung besteht daher darin, daß Flach- oder Kapillarmembranen der oben bezeichneten Art bereitgestellt werden, die dadurch gekennzeichnet sind, daß sie aus 70 bis 98 Gewichtsprozent Polyvinylidenfluorid und 2 bis 30 Gewichtsprozent aus einem im wesentlichen aus Polyacrylsäuremethyl- und/oder -ethylester gebildeten Polymeren bestehen und eine maximale Porengröße im Bereich von 0,005 bis 10 »m aufweisen. Bevorzugte Bereiche für die maximale Porengröße stellen die von 0,01 bis 2 »m und von 0,05 bis 0,8 »m dar. Der maximale Porendurchmesser wird mittels der Blaspunktmethode nach ASTM Nr. 128-61 und F 316-70 bestimmt.

Sowohl das mittlere Molekulargewicht des Polyvinylidenfluorids als auch das mittlere Molekulargewicht des im wesentlichen aus Polyacrylsäuremethyl- und/oder -ethylester gebildeten Polymeren können in weiten Grenzen variieren. Unter dem mittleren Molekulargewicht wird hierin das Gewichtsmittel M_{w}, gemessen durch Gelpermeationschromatographie nach vorheriger Eichung mit einer entsprechenden Standard-Polymerlösung, verstanden. Das für die weiter unten beschriebenen zwei erfindungsgemäßen Verfahren zur Herstellung der Flach- oder Kapillarmembranen verwendete Polyvinylidenfluorid kann im allgemeinen ein mittleres Molekulargewicht von 30 000 bis 500 000 aufweisen, ein mittleres Molekulargewicht von 50 000 bis 500 000 wird hierbei bevorzugt. Ebenso kann das mittlere Molekulargewicht des im wesentlichen aus Polyacrylsäuremethyl- und/oder -ethylester gebildeten Polymeren zwischen 5 000 und 1 000 000 variieren, wenngleich für die Herstellung der Flach- oder Kapillarmembranen ein mittleres Molekulargewicht im Bereich von 50 000 bis 200 000 bevorzugt wird.

Die erfindungsgemäßen Flach- oder Kapillarmembranen können in der Weise hergestellt werden, daß man, bezogen auf das Polymergesamtgewicht, aus 70 bis 98 Gewichtsprozent Polyvinylidenfluorid und 2 bis 30 Gewichtsprozent eines im wesentlichen aus Polyacrylsäuremethyl- und/oder -ethylester gebildeten Polymeren unter Verwendung von einem oder mehreren Lösungsmitteln und einem oder mehreren Nichtlösern eine 10 bis 40 Gew.-%ige Lösung, bezogen auf deren Gesamtgewicht, herstellt, die oberhalb Raumtemperatur im flüssigen Zustand einen Bereich völliger Mischbarkeit und eine Mischungslücke aufweist und oberhalb Raumtemperatur einen Erstarrungsbereich besitzt, indem man die Stoffkomponenten unter intensivem homogenen Mischen auf eine Temperatur oberhalb der Mischungslücke aufheizt, die so erhaltene Lösung von der Temperatur oberhalb der Mischungslücke in einer Abkühlflüssigkeit schnell abkühlt und gleichzeitig zu einer Flach- oder Kapillarmembran ausformt und anschließend die Membran durch Extraktion von Lösungsmittel -und Nichtlöserresten befreit.

Diese Verfahrensweise lehnt sich eng an das in der DE-A-33 29 578 beschriebene und vom Patentanmelder stammende Verfahren zur Herstellung von Poren aufweisenden Polyvinylidenfluorid-Formkörpern an, auf deren Inhalt hier ausdrücklich verwiesen wird. Zur Herstellung der Lösung werden die Polymeren bei erhöhter Temperatur, vorzugsweise bei 200 bis 230°C, in einem Gemisch von mindestens einem Lösungsmittel und mindestens einem Nichtlöser aufgelöst. Unter Lösungsmitteln sind im Rahmen der Erfindung auch Flüssigkeiten zu verstehen, die bei Raumtemperatur die Polymere nicht oder nur sehr schlecht lösen, die aber bei erhöhter Temperatur gute Löseeigenschaften aufweisen. Geeignete Lösungsmittel für die Polymeren sind Glycerintriacetat, Glycerindiacetat, 2-(2-Butoxyäthoxy-)ethylacetat, ε-Caprolactam und Gemische aus den erwähnten Verbindungen. Die Verwendung von Glycerintriacetat als Lösungsmittel oder eines Gemisches aus Glycerintriacetat und ε-Caprolactam wird bevorzugt. Als Nichtlöser sind Di-n-octyladipat oder Rizinus-Öl oder ein Gemisch hiervon gut geeignet. Die bei erhöhter Temperatur hergestellte homogene 10 bis 40 gew.-%ige, vorzugsweise 20 bis 30 gew.-%ige Lösung, bezogen auf deren Gesamtgewicht, enthält, bezogen auf das Polymergesamtgewicht, vorzugsweise 80 bis 95 Gewichtsprozent Polyvinylidenfluorid und 5 bis 20 Gewichtsprozent Polyacrylsäuremethyl- und/oder - ethylester. Das mittlere Molekulargewicht des PVDF sollte vorzugsweise 50 000 bis 500 000 und das von Polyacrylsäuremethyl- und/oder -ethylester vorzugsweise 50 000 bis 200 000 betragen.

Die besagten homogenen Lösungen werden zu einer Kapillarmembran oder zu einer Flachmembran ausgeformt und dabei schnell abgekühlt, wobei sie zuerst einen Bereich der Phasentrennung durchlaufen. Die eine der beiden nach der Entmischung gebildeten flüssigen Phasen stellt eine an Polymeren verarmte flüssige Phase aus Lösungsmittel(n) und Nichtlöser(n) dar, die andere eine an Lösungsmittel(n) und Nichtlöser(n) verarmte und mit den Polymeren angereicherte flüssige Phase. Letztere führt bei weiterer Abkühlung zur Verfestigung der Polymeren. Für die Abkühlung ist es vorteilhaft, als Abkühlungsflüssigkeit Wasser, gegebenenfalls mit einem Zusatz an Tensid, zu verwenden.

Es ist aber auch möglich, als Lösungsmittel eine Flüssigkeit oder ein Flüssigkeitsgemisch zu verwenden, die bei Raumtemperatur oder nur leicht erhöhter Temperatur, also im allgemeinen in einem Temperaturbereich von 15 bis 50°C, eine klare Lösung ergeben. Als Lösungsmittel kommen insbesondere die aprotischen Lösungsmittel in Frage. In diesem Fall wird vorzugsweise aus einer oder mehreren Verbindungen aus der Gruppe von N-Methylpyrrolidon, Dimethylsulfoxid, Dioxan, Dimethylformamid und Dimethylacetamid bei einer Temperatur von 0 bis 80°C, insbesondere 20 bis 40°C, eine 10 bis 40 Gew.-%ige Lösung, bezogen auf deren Gesamtgewicht, hergestellt und die Lösung nach dem Ausformen als Kapillarmembran oder als Flachmembran durch Eintauchen in einen Nichtlöser ausgefällt, wobei die Temperatur des Nichtlöserbades 0 bis 80°C, insbesondere 20 bis 40°C, beträgt. Als Nichtlöser kommen alle Flüssigkeiten in Betracht, die die Polymeren bei Raumtemperatur nicht lösen und mit dem Lösungsmittel der Polymerlösung zumindest in begrenztem Umfang mischbar sind. Zur Koagulation der Membran werden als Nichtlöser Alkohole mit 1 bis 12 C-Atomen, insbesondere mit 1 bis 3 C-Atomen, Wasser oder Gemische der genannten Stoffe bevorzugt.

Es ist in machen Fällen günstig, die Lösung vor ihrem Kontakt mit dem flüssigen Nichtlöser für eine gewisse Zeit, die von einigen Sekunden bis zu einigen Minuten reichen kann, in Kontakt mit gasförmigem Nichtlöser, wie feuchter Luft, Wasserdampf oder Dämpfen der vorerwähnten Alkohole, zu bringen. Es kann auch vorteilhaft sein, der zur Membranherstellung eingesetzten Polymerlösung bestimmte Additive zuzusetzen. In Frage kommen zum Beispiel Verdickungsmittel zum Erhöhen der Viskosität der Polymerlösung oder Nukleierungsmittel zur Beeinflussung des Membranbildungsprozesses oder Farbstoffe oder Pigmente.

Die nach einem der beschriebenen Verfahren hergestellte Membran wird extrahiert, um Reste von Lösungsmitteln und sonstige Stoffe zu entfernen, die bei einer späteren Anwendung der Membran stören würden. Als Extraktionsmittel können alle Fluide eingesetzt werden, die die zu extrahierenden Stoffe, jedoch nicht die Membranpolymeren PVDF, Polyacrylsäuremethylester und/oder Polyacrylsäureethylester lösen. Bevorzugt werden niedere Alkohole mit 1 bis 3 C-Atomen, insbesondere Isopropanol, verwendet. Das Extraktionsmittel wird durch Trocknen aus der Membran entfernt.

Die zur Herstellung der Flach- oder Kapillarmembran erfindungsgemäß im wesentlichen verwendeten Polyacrylsäuremethyl- und/oder - ethylester eines mittleren Molekulargewichts von 5 000 bis 1000 000 sind bekanntlich nicht kommerziell erhältlich. Sie können aber nach bekannten Verfahren sogar in einem Lösungsmittel, wie Triacetin (= Glycerin-triacetat), N-Methylpyrrolidon oder Dimethylsulfoxid, das auch als Lösungsmittel bei der Membranherstellung eingesetzt wird, synthetisiert werden, indem man diesem bei Raumtemperatur eine Menge von 1 bis 30 Gewichtsprozent Acrylsäuremethyl- und/oder -ethylester und 0,02 bis 5 Gewichtsprozent, bezogen auf die Monomeren, eines Radikalstarters, wie Benzoylperoxid, Azobisisobutyronitril oder Acetovaleronitril, zusetzt. Entsprechend wird bei der Mischpolymerisation der Acrylsäuremethyl- und/oder -ethylester-Monomeren mit geringen Mengen der oben beispielhaft genannten Monomeren verfahren. Anschließend wird die Lösung auf 80°C hochgeheizt, um die Polymerisation einzuleiten. Nach der Polymerisation werden nicht umgesetzte Monomere bei erhöhter Temperatur und unter Zuhilfenahme eines Schleppmittels, wie Wasser, niedere Alkohole oder Essigsäureethylester, ausgetrieben. Die Lösung sollte keine Monomeren mehr enthalten, um bei der Membranherstellung das Personal nicht durch die toxischen Monomeren zu gefährden und um Restgehalte der Monomeren in der fertigen Membran zuverlässig zu vermeiden.

Die so erhaltene Polyacrylsäuremethyl- und/oder -ethylester-Lösung wird für die oben beschriebene Membranherstellung nach der DE-A-33 29 578 nur noch mit den entsprechenden Mengen an Nichtlösern und Polyvinylidenfluorid versetzt und zur Erzielung einer homogenen Lösung unter Rühren aufgeheizt.

Die erfindungsgemäße hydrophobe Membran gemäß Anspruch 1 ist mechanisch und thermisch stabil und weist gegen den Angriff von Oxidationsmitteln und Säuren eine hohe chemische Beständigkeit auf. Überraschend wurde gefunden, daß sie gegenüber Natronlauge eine erheblich bessere chemische Beständigkeit besitzt als die hydrophoben PVDF-Membranen des Standes der Technik. So tritt die erste Verfärbung von üblichen PVDF-Membranen in 10%-iger Natronlauge bei 40°C bereits nach 5 Minuten auf, wogegen die erfindungsgemäß modifizierten Membranen erst zwischen 40 und 60 Minuten erste Verfärbungserscheinungen zeigen. Die Beständigkeit von Membranen gegen Alkalien ist in der Praxis bei der Filtration basischer Medien oder bei der Reinigung der Membran mit Natronlauge von großer Bedeutung.

Die erfindungsgemäße hydrophobe Membran gemäß Anspruch 1 zeichnet sich überraschenderweise gegenüber PVDF-Membranen bei gleicher Porengröße durch eine höhere Porosität der Außenoberfläche aus. Unter Porosität der Außenoberfläche wird die Fläche an offenen Poren an der Außenoberfläche der Membran im Verhältnis zur Außenoberfläche verstanden. Die Porosität der Außenoberfläche ist von entscheidender Bedeutung für das Verstopfen einer Membran. Je größer diese Porosität einer Membran ist, desto langsamer wird sie bei sachgemäßer Benutzung verstopft.

Die neue hydrophobe Membran eignet sich daher hervorragend für die Filtration von Gasen oder für Anwendungen, bei denen eine hydrophile Flüssigkeit die Membran nicht passieren darf. Beispiele dafür stellen die Begasung von Flüssigkeiten (Blut, Bioreaktoren, Abwasser) oder die Transmembrandestillation dar.

Die erfindungsgemäße hydrophobe Membran gemäß Anspruch 1 kann insbesondere an ihrer gesamten Oberfläche einer chemischen Modifizierung zwecks Erhalts einer hydrophilen Membran unterworfen werden, indem die Estergruppen auf der Gesamtoberfläche der Membran einer zumindest teilweisen Hydrolyse und/oder einer zumindest teilweisen Umesterung mit einem mehrwertigen Alkohol- und/oder einer zumindest teilweisen Aminolyse mit einer Aminoverbindung mit 2 - 8 C-Atomen unterworfen werden.

Unter Gesamtoberfläche wird hierin nicht nur die äußere Oberfläche verstanden, sondern auch die inneren Oberflächen, d.h. auch die Oberflächen der Microporen der Membran, die während ihres Gebrauchs durch Fluide kontaktiert werden. Die hydrophilierten Flach- oder Kapillarmembranen sind dann dadurch gekennzeichnet, daß sie auf ihrer gesamten Oberfläche 0,001 bis 10 mVal/g Membran, vorzugsweise 0,01 bis 5 mVal/g Membran -OH, -NH₂ oder -COOH-Gruppen oder Gemische dieser hydrophilen funktionellen Gruppen enthalten.

Die permanente Hydrophilierung der erfindungsgemäßen Membran ist, wie bereits oben erwähnt, deshalb so überraschend, weil sich Membranen, die neben PVDF noch bis zu 30 Gew.-% Ester der Acrylsäure mit einwertigen Alkoholen einer höheren Kohlenstoffatomzahl als 2 oder Ester der Methacrylsäure mit einwertigen Alkoholen enthalten, nach der geschilderten Verfahrensweise nicht hydrophilieren lassen.

Eine nur teilweise Hydrolyse oder teilweise Umesterung oder teilweise Aminolyse der erfindungsgemäßen Membran gemäß Anspruch 1 kommt insbesondere dann in Betracht, wenn die Membran vergleichsweise größere Mengen an Polyacrylsäuremethyl- und/oder - ethylester, beispielsweise 10 oder 20 Gew.-%, enthält. Teilweise Umsetzungen der geschilderten Art können aber auch dann erfindungsgemäß in Betracht kommen, wenn die Membran zwar hydrophile Gruppen enthält, aber hydrophob bleiben soll, oder wenn die hydrophilierte Membran Gemische der oben genannten hydrophilen funktionellen Gruppen enthalten soll. Für diesen Zweck kann die Reihenfolge der betreffenden chemischen Umsetzungen beliebig gewählt werden.

Bezüglich der Durchführbarkeit der betreffenden chemischen Umsetzungen wird angenommen, daß die Polyacrylat-Makromoleküle statistisch im PVDF verteilt sind. Dadurch sind auf der gesamten Oberfläche im Inneren der Poren der Membran vereinzelt Teile von Makromolekülen des Polyacrylsäuremethyl- und/oder - ethylesters anzutreffen. Die Estergruppen dieser Makromolekülteile können durch die betreffenden chemischen Umsetzungen zumindest partiell in -OH, -NH₂ oder -COOH-Gruppen überführt werden, während die entsprechenden Teile der Polyacrylat-Makromoleküle im Inneren der Membran keine chemische Umsetzung erleiden können. Dadurch bleiben diese in der Membranstruktur verankert und können nicht ausgewaschen werden. Die hydrophilen funktionellen Gruppen in den Makromolekülteilen des an den äußeren und inneren Oberflächen der Membran vorliegenden Acrylsäurepolymerisats hydrophilieren dagegen die Membran permanent.

Die Hydrolyse der erfindungsgemäßen Membran gemäß Anspruch 1 kann in der Weise durchgeführt werden, daß man die Estergruppen auf der Gesamtoberfläche der Membran mit konzentrierter Schwefelsäure bei einer Temperatur von 40 bis 80°C 1 bis 20 Stunden lang behandelt. Die Hydrolysegeschwindigkeit nimmt hierbei mit steigender Temperatur zu. Wenn auch andere starke Säuren zur Hydrolyse herangezogen werden können, wie Chlorwasserstoffsäure, Methansulfonsäure und Perchlorsäure, so wird im Rahmen der Erfindung eine Behandlung der Membran mit konzentrierter Schwefelsäure bevorzugt. Um die Membran bei dieser Behandlung benetzen zu können und um die Temperaturerhöhung durch die Verdünnungswärme der Schwefelsäure hinreichend klein zu halten, kann es vorteilhaft sein, die Membran nacheinander z.B. in folgende Lösungen zu tauchen:
C₁-C₄-Alkohol - Wasser - 50%-ige H₂SO₄ - 70%-ige H₂SO₄ - 98%-ige H₂SO₄ - 70%-ige H₂SO₄ - 50%-ige H₂SO₄ - Wasser.

Die Hydrolyse kann im vorliegenden Fall auch unter basischen Bedingungen durchgeführt werden. Hierbei ist allerdings zu beachten, daß der p_{H}-Wert des Reaktionsmediums < 11 sein sollte, da ansonsten das Polyvinylidenflourid angegriffen wird. Zu diesem Zweck ist es günstig, in Gegenwart einer Pufferlösung zu arbeiten, als die sich eine bei Raumtemperatur gesättigte wäßrige Boraxlösung als besonders geeignet erwies. Da auch die basische Hydrolyse relativ langwierig ist, arbeitet man vorteilhafterweise in einem Druckgefäß bei einer Temperatur von 80 bis 140°C und einem pH-Wert von 9 bis 11.

Die erfindungsgemäße Membran gemäß Anspruch 1 kann auch hydrophiliert werden, indem man sie mittels einer Umesterung (= Alkoholyse) auf ihrer gesamten Oberfläche mit alkoholischen OH-Gruppen versieht. Zu diesem Zweck werden die Estergruppen auf der Gesamtoberfläche der Membran mit einem mindestens dreiwertigen Alkohol unter Zusatz von 0,1 bis 10 Gew.-%, bezogen auf den mehrwertigen Alkohol, einer starken Säure bei einer Temperatur von 100 bis 150°C 1 bis 30 Stunden lang einer zumindest teilweisen Umesterung unterworfen. Als starke Säuren können hierbei beispielsweise Schwefelsäure, Chlorwasserstoffsäure, Methansulfonsäure und Perchlorsäure verwendet werden.

Als mehrwertige Alkohole eignen sich Alkohole mit drei und mehr OH-Gruppen, wie Glycerin, Diglycerin, Triglycerin, Polyglyceringemische, Trimethyloläthan, Trimethylolpropan, Pentaerythrit, Dipentaerythrit, Tripentaerythit, Butantriol-(1.2.4), Hexantriol, Zuckeralkohole, wie Sorbit und Mannit, und Monosaccharide, wie Fructose und Mannose. Selbstverständlich können auch Gemische von mehrwertigen Alkoholen verwendet werden. Bevorzugt werden als mehrwertiger Alkohol eine oder mehrere Verbindungen aus der Gruppe von Glycerin, Diglycerin, Triglycerin, einem Polyglyceringemisch, Pentaerythrit und Sorbit verwendet.

Erstaunlicherweise führt eine analoge Behandlung der Membran in Ethylenglykol oder niedermolekularen Polyethylenglycolen nicht zu einer Hydrophilierung der Membranoberfläche. Ethylenglycol kann aber vorteilhafterweise als Reaktionsmedium für die Alkoholyse verwendet werden, um die in Betracht kommenden festen Stoffe, wie Pentaerythrit und die Monosaccharide, zu lösen und mit der Membran in Reaktion zu bringen.

Eine weitere Verfahrensweise zur Hydrophilierung der erfindungsgemäßen Membran gemäß Anspruch 1 stellt die Aminolyse dar. Da primäre und sekundäre Amine wegen ihrer Basizität Polyvinylidenfluorid bei erhöhter Temperatur angreifen, sind die Reaktionsbedingungen, wie p_{H}-Wert, Temperatur und Reaktionszeit, dieser Gegebenheit anzupassen. Als günstig hat es sich erwiesen, daß man die Estergruppen auf der Gesamtoberfläche der Membran mit mindestens einer Aminoverbindung mit 2 bis 8 C-Atomen unter Verwendung von entsprechenden Puffergemischen bei einem p_{H}-Wert < 11 und einer Temperatur von 50 bis 150°C ein bis 24 Stunden lang einer zumindest teilweisen Aminolyse unterwirft. Im einfachsten Fall kann hierbei die Lösung der entsprechenden Aminoverbindung bis zur Sättigung mit Ammoniumchlorid abgepuffert werden.

Unter Aminoverbindung im Sinne der Erfindung werden hierin organische Verbindungen mit 2 bis 8 C-Atomen mit einer oder mehreren primären Aminogruppen verstanden, mit der Maßgabe, daß bei Anwesenheit von nur einer primären Aminogruppe mindestens noch zwei weitere hydrophile funktionielle Gruppen in Form von Hydroxyl- und/oder Carboxylgruppen vorliegen. Beispiele hierfür sind 1,2-Diaminoäthan, 1,2-Diaminopropan, 1,3-Diaminopropan, 1,4-Diaminobutan, 1,3-Diaminobutan, 1,5-Diaminopentan, 1,8-Diaminooctan, Asparaginsäure, Glutaminsäure und Homo-serin.

Eingeschlossen sind hierin insbesondere auch primäre und sekundäre Polyamine mit 2 primären Aminogruppen sowie Amine mit 3 primären Aminogruppen, spezifische Harnstoffderivate und heterocyclische Hydrazine mit mehreren Hydrazinresten. Wie im Falle der oben beschriebenen Alkoholyse erfordert dann die erfindungsgemäße Umwandlung der hydrophoben Membran in eine hydrophile Membran wegen der höheren Anzahl an hydrophilen Gruppen pro Molekül nur die Anwesenheit von vergleichsweise niedrigen Gewichtsmengen an Polyacrylsäuremethyl- und/oder - ethylester in der Flach- oder Kapillarmembran gemäß Anspruch 1, was den Gebrauchseigenschaften der hydrophilen Membran zugute kommt. Beispiele hierfür sind Diäthylentriamin, Triäthylen tetramin, Tetraäthylenpentamin, Dipropylentriamin, 1,2,3-Triaminopropan, 2,4,6-Triamino-1,3,5-triazin, 2,4,6-Trihydrazino-1,3,5-triazin, Isobutylidendiharnstoff, Biuret und Triuret. Selbstverständlich können auch Gemische von verwendbaren Aminoverbindungen verwendet werden. Es wird bevorzugt, daß als Aminoverbindung eine oder mehrere Verbindungen aus der Gruppe von Diäthylentriamin, Triäthylentetramin und Tetraäthylenpentamin verwendet werden.

Überraschend wurde gefunden, daß auch die in der oben beschriebenen Art und Weise hydrophilierten Flach- oder Kapillarmembranen gegenüber Natronlauge eine erheblich bessere chemische Beständigkeit besitzen als die hydrophoben PVDF-Membranen des Standes der Technik. Während die erste Verfärbung von üblichen PVDF-Membranen in 10%-iger Natronlauge bei 40°C bereits nach 5 Minuten auftritt, zeigen die erfindungsgemäß hydrophilierten Membranen erst nach 45 Minuten erste Verfärbungserscheinungen. Diese überraschende Eigenschaft gestattet daher extreme Anwendungsbedingungen bei der Verwendung der erfindungsgemäßen hydrophilen Flach-oder Kapillarmembran zur Immobilisierung biochemisch aktiver Verbindungen, die bei den hydrophilen Membranen des Standes der Technik ausgeschlossen sind. Unter biochemisch aktiven Verbindungen werden hierin Substrate, Inhibitoren und Coenzyme von Enzymen sowie deren Analoga, Enzyme selbst, sonstige Proteine, sonstige Zellbestandteile, ganze Zellen oder von Zellen produzierte Verbindungen, sowie Verbindungen, welche mit den aufgeführten Stoffgruppen, also auch mit ganzen Zellen, in irgendeiner Weise in Wechselwirkung treten können, verstanden.

Die in den Ansprüchen 7 bis 8 beschriebenen hydrophilen Membranen besitzen OH-, NH₂- oder COOH-Endgruppen oder Gemische dieser hydrophilen funktionellen Endgruppen. Derartige Membranen lassen sich in vorteilhafter Weise mit biochemisch aktiven Verbindungen umsetzen. Derartige Umsetzungen können Reaktionen von NH₂-Gruppen mit Aldehyden, reaktiven Carbonsäurederivaten und Alkyl- bzw. Arylhalogeniden, von OH-Gruppen mit reaktiven Carbonsäurederivaten und Alkyl- oder Arylhalogeniden sowie von (aktivierten) COOH-Gruppen mit Aminen, Alkoholen und Alkyl- bzw. Arylhalogeniden als Anfangsschritt einschließen. Eine Reihe solcher Umsetzungen wird z.B. in der DE-A-28 28 194 für die Derivatisierung einer Matrix auf Polysaccharid-Basis beschrieben. Wegen der Komplexität dieses Gebietes können die hier und in der DE-A-28 28 194 aufgeführten Reaktionen jedoch nicht umfassend sein. Eine geeignete chemische Umsetzung ist immer auf den zu bearbeitenden Einzelfall abzustimmen, da die Natur der bearbeiteten biologisch aktiven Verbindungen in erheblichem Maße schwanken kann und auch die Natur der Matrix Einfluß auf das Ergebnis haben kann. Werden solche Umsetzungen an der erfindungsgemäßen hydrophilen Membran durchgeführt, so wird eine aktivierte Membran erhalten, die ihrerseits leicht mit anderen, funktionelle Gruppen aufweisenden Molekülen reagieren kann. Zum Unterschied dazu wird eine Membran, an der ein biochemisch aktives Molekül, ein Zellbestandteil oder eine Zelle gebunden ist, hierin als derivatisierte Membran bezeichnet.

Die biochemisch aktive Verbindung kann zuweilen direkt an der Membran oder, falls z.B. sterische Hinderungen dies nicht zulassen, auch nach Zwischenschaltung von einem oder mehreren Spacern immobilisiert werden. Unter einem Spacer wird hierin ein Molekül verstanden, welches mindestens zwei funktionelle Gruppen, z.B. in Form einer Aldehydgruppe, Aminogruppe, Carboxylgruppe oder Hydroxylgruppe, enthält und als bleibender "Abstandshalter" wirkt.

Die erfindungsgemäße Verwendung der hydrophilen Flach- oder Kapillarmembran zur Immobilisierung biochemisch aktiver Verbindungen führt zu einer derivatisierten Membran, die u.a. als Medium für eine Kombination von Filtration und Affinitätschromatographie Verwendung finden kann. Hierfür werden z.B. Substrat(e), Coenzym(e), Inhibitor(en), Antikörper und/oder deren Analoga als kovalent gebundene Liganden eingesetzt. Weiterhin eignet sich die chemisch aktivierte Membran zur Immobilisierung von Enzymen, sonstigen Proteinen, Zellbestandteilen und/oder ganzen Zellen. Hierbei verbindet sich der Vorteil einer Filtrationseinheit mit denen literaturbekannter Immobilisierungen.

Die in Rede stehenden derivatisierten Membranen besitzen die besonders vorteilhafte Eigenschaft, eine chemisch und physikalisch äußerst stabile, im allgemeinen hydrophile Grundstruktur aufzuweisen. Dies ist insbesondere für die chemische Aktivierung unter drastischen Bedingungen (extreme p_{H}-Werte, höhere Temperatur, aggressives Lösungsmittel) ein entscheidender Vorteil. Bei der Reinigung von gebrauchter derivatisierter Membran stellt sich darüber hinaus der folgende überraschende Effekt ein. So kann, wie das in Beispiel 24 ausgeführt wird, bei Verwendung von chemisch relativ beständigen Liganden nicht nur mit starken Säuren, sondern auch mit starken Laugen wesentlich schneller und effektiver gereinigt werden als beispielsweise bei den bekannten Trägermaterialien cellulosischer Natur, ohne daß ein Abbau der Membran oder des Liganden stattfindet. Mit den bekannten Membranen auf Cellulose-Basis wäre ein derartiges Vorgehen undenkbar, da sie unter diesen Bedingungen sehr stark geschädigt bzw. zerstört werden würden.

Ferner kann sogar eine etwas weniger beständige Bindung zwischen der Membran und dem Ligand unter entsprechend drastischen Bedingungen, wie z.B. den in den Beispielen 10, 16 und 23 angeführten, gespalten werden und damit wird die Membran in ihren hydrophilen Urzustand zurückversetzt. Die Membran ist somit mehrmals zur Immobilisierung biochemisch aktiver Verbindungen verwendbar, was bisher noch nicht für eine derivatisierbare Membran beschrieben worden ist. Dies ist insbesondere für die Immobilisierung von Enzymen mit geringer Lebensdauer von Vorteil.

Die vorliegende Erfindung wird anhand der nachfolgenden Beispiele näher erläutert:

### A. Herstellung der Polyacrylsäureester

- A₁:: Herstellung von Polyacrylsäuremethylester (= PMA), M_{w} = 7 400
In einem beheizbaren 4 l Glaskolben werden 1 800 g Triacetin bei 70°C vorgelegt. Die Flüssigkeit wird ca. 0,5 h mit Stickstoff durchströmt, um Sauerstoff zu entfernen.
In 200 g Methylacrylat (MA) werden bei Raumtemperatur 4 Gewichtsprozent Acetovaleronitril gelöst. Diese Lösung wird innerhalb einer Stunde in den Glaskolben mit dem Triacetin getropft. Eine halbe Stunde nach Ende der Zugabe wird das Reaktionsgemisch für eine halbe Stunde auf 160°C erhitzt, um restliche Monomere weitgehend umzusetzen. Danach werden durch Destillation von ca. 100 g Triacetin die letzten Reste an Monomeren aus der Lösung entfernt. Zurück bleibt eine viskose Lösung von Polymethylacrylat (PMA) in Triacetin. Das mittlere Molekulargewicht betrug 7 400.
- A₂:: Herstellung von PMA, M_{w} = 35 000
In einem beheizbaren 4 l Glaskolben werden 1 400 g Triacetin (= Glycerin-triacetat) bei 70°C vorgelegt. Die Flüssigkeit wird ca. 0,5 h mit Stickstoff durchströmt, um Sauerstoff zu entfernen.
In 600 g Methylacrylat (MA) werden bei Raumtemperatur 5 Gewichtsprozent Benzoylperoxid gelöst. Diese Lösung wird innerhalb einer Stunde in den Glaskolben mit dem Triacetin getropft. Eine halbe Stunde nach Ende der Zugabe wird das Reaktionsgemisch für eine halbe Stunde auf 160°C erhitzt, um restliche Monomere weitgehend umzusetzen. Danach werden durch Destillation von ca. 100 g Triacetin die letzten Reste an Monomeren aus der Lösung entfernt. Zurück bleibt eine viskose Lösung von Polymethylacrylat (PMA) in Triacetin. Das mittlere Molekulargewicht M_{w} (Gewichtsmittelwert) betrug 35 000.
- A₃:: Herstellung von PMA, M_{w} = 235 000
In einem beheizbaren 4 l Glaskolben werden 1 400 g Triacetin bei 70°C vorgelegt. Die Flüssigkeit wird ca. 0,5 h mit Stickstoff durchströmt, um Sauerstoff zu entfernen.
In 600 g Methylacrylat (MA) werden bei Raumtemperatur 3 Gewichtsprozent Benzoylperoxid gelöst. Diese Lösung wird innerhalb einer Stunde in den Glaskolben mit dem Triacetin getropft. Eine halbe Stunde nach Ende der Zugabe wird das Reaktionsgemisch für eine halbe Stunde auf 160°C erhitzt, um restliche Monomere weitgehend umzusetzen. Danach werden durch Destillation von ca. 100 g Triacetin die letzten Reste an Monomeren aus der Lösung entfernt. Zurück bleibt eine viskose Lösung von Polymethylacrylat (PMA) in Triacetin. Das mittlere Molekulargewicht betrug 230 000.
- A₄:: Herstellung von PMA, M_{w} = 688 000
In 20 g Methylacrylat (MA) werden bei Raumtemperatur 0,5 Gewichtsprozent Acetovaleronitril gelöst. Diese Lösung wird innerhalb einer Stunde in einem Glaskolben auf 80°C aufgewärmt. Nach einer weiteren halben Stunde wird das Reaktionsgemisch für eine halbe Stunde auf 160°C erhitzt, um restliche Monomere weitgehend umzusetzen. Nach Abkühlen erhält man ein gummiartiges klares Polymer. Das mittlere Molekulargewicht betrug 688 000.
- A₅:: Herstellung von Polyacrylsäureethylester (= PEA), M_{w} = 114 000
In einem beheizbaren 4 l Glaskolben werden 1600 g Triacetin bei 70°C vorgelegt. Die Flüssigkeit wird ca. 0,5 h mit Stickstoff durchströmt, um Sauerstoff zu entfernen.
In 400 g Ethylacrylat (EA) werden bei Raumtemperatur 0,2 Gewichtsprozent Benzoylperoxid gelöst. Diese Lösung wird innerhalb einer Stunde in den Glaskolben mit dem Triacetin getropft. Eine halbe Stunde nach Ende der Zugabe wird das Reaktionsgemisch für eine halbe Stunde auf 160°C erhitzt, um restliche Monomere weitgehend umzusetzen. Danach werden durch Destillation von ca. 100 g Triacetin die letzten Reste an Monomeren aus der Lösung entfernt. Zurück bleibt eine viskose Lösung von Polyethylacrylat (PEA) in Triacetin. Das mittlere Molekulargewicht M_{w} betrug 114 000.
- A₆:: Herstellung von PMA/PEA, M_{w} = 160 000
In einem beheizbaren 2 l Glaskolben werden 800 g Triacetin bei 70°C vorgelegt. Die Flüssigkeit wird ca. 0,5 h mit Stickstoff durchströmt, um Sauerstoff zu entfernen.
In 150 g Methylacrylat (MA) und 150 g Ethylacrylat (EA) werden bei Raumtemperatur 0,2 Gewichtsprozent Benzoylperoxid gelöst. Diese Lösung wird innerhalb einer Stunde in den Glaskolben mit dem Triacetin getropft. Eine halbe Stunde nach Ende der Zugabe wird das Reaktionsgemisch für eine halbe Stunde auf 160°C erhitzt, um restliche Monomere weitgehend umzusetzen. Danach werden durch Destillation von ca. 100 g Triacetin die letzten Reste an Monomeren aus der Lösung entfernt. Zurück bleibt eine viskose Lösung eines Copolymeren aus Methylacrylat (MA) und Ethylacrylat (EA) in Triacetin. Das mittlere Molekulargewicht Mw betrug 160 000.

### B. Herstellung der erfindungsgemäßen Flach- oder Kapillarmembranen.

### Beispiel 1:

### Herstellung einer PVDF/PMA-Flachmembran.

In einem Glaskolben mit Rührer wurden 27 Teile (stets Gewichtsteile) PVDF der Type Kynar 460 (Pennwalt), 3 Teile Polymethylacrylat (A₁), 18,2 Teile Triacetin, 4,55 Teile ε-Caprolactam und 47,25 Teile Dioctyladipat zusammengegeben. Das bei diesem und den weiteren Versuchen, mit Ausnahme von Beispiel 2, eingesetzte PVDF hat, soweit nicht anders angegeben, nach der Gelpermeationschromatographie ein mittleres Molekulargewicht von 361 000. Unter Rühren wurde die Mischung auf 240°C aufgeheizt, bis man eine homogene Lösung erhielt. Danach wurde die Lösung auf 190°C abgekühlt. Diese Lösung wurde auf einer Gießwalze, die auf 20°C temperiert war, mit Hilfe eines Gießkastens zu einer Folie ausgeformt und auf einer Walze aufgewickelt. Proben der Folien wurden 3 mal für 0,5 h in Isopropanol bei 60°C extrahiert, um die Lösungsmittel weitestgehend aus der Membran auszuwaschen. Nach dem Trocknen erhielt man eine weiße Flachmembran.

Die Membrandicke beträgt 120 »m, die maximale Porengröße der Membran 0,55 »m. Der Isopropanolfluß der Membran beträgt 7,4 ml/(cm² min bar).

Bei einem Transmembrandruck von 0,5 bar findet kein Wasserfluß durch die Membran statt.

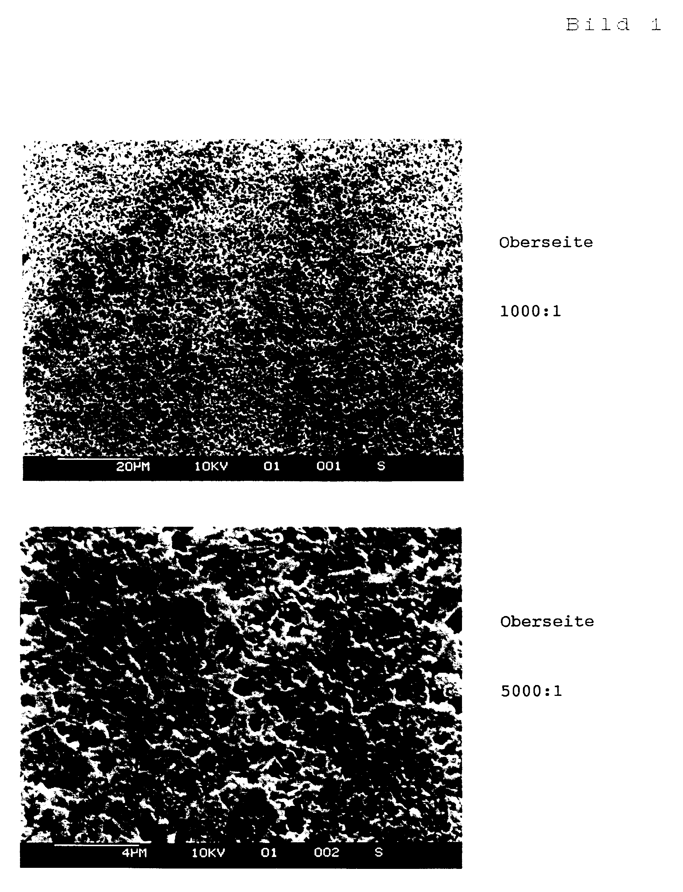

Die Oberflächen der Flachmembran sind sehr offenporig (vgl. Mikrofotografie, Bild 1).

### Beispiel 2:

### Herstellung einer PVDF/PMA-Flachmembran mit einem niedrigen M_{w} von PVDF.

In einem Glaskolben mit Rührer wurden 56 g PVDF der Type Kynar 740 (Pennwalt) (M_{w} = 180 000), 132 g Dioctyladipat, 12 g Triacetin und 75 g einer 20 prozentigen Lösung von Polyethylacrylat (A₅) in Triacetin zusammengegeben. Unter Rühren wurde die Mischung auf 240°C aufgeheizt, bis man eine homogene Lösung erhielt. Diese Lösung wurde auf einer Glasplatte zu einer Folie von 200 »m Schichtdicke ausgerakelt. Durch Eintauchen in Wasser wurde die Folie zum Erstarren gebracht. Die Folie wurde 3 mal für 0,5 h in Isopropanol bei 60°C extrahiert, um die Lösungsmittel weitestgehend aus der Membran auszuwaschen. Nach dem Trocknen erhielt man eine weiße Flachmembran.

Die maximale Porengröße der Membran betrug 1,3 »m.

### Beispiel 3:

### Herstellung von PVDF/PMA Kapillarmembranen mit sehr kleinen Poren.

Eine analog zu Beispiel 1 hergestellte Lösung von 32,5 Teilen PVDF der Type Kynar 460, 2,5 Teilen PMA (A₂), 22,75 Teilen Triacetin und 42,25 Teilen Dioctyladipat wurde bei einer Temperatur von 205°C durch eine Ringspaltdüse zu einer Kapillarmembran versponnen. Zur Offenhaltung des Kapillarhohlraumes wurde durch eine Hohlnadel im Innern der Ringspaltdüse Glycerin gefördert.

Die Membran wurde 3 mal eine halbe Stunde in Isopropanol bei 60°C extrahiert.

Die Kapillarmembran hatte einen Innendurchmesser von 0,2 mm und einen Außendurchmesser von 0,29 mm.

Die maximale Porengröße der Membran war kleiner als ca. 0,2 »m und somit nicht nach der Blaspunktmethode bestimmbar. Der Isopropanolfluß betrug 0,15 ml/(cm² min bar).

Die Trenneigenschaften der Membran wurden durch Bestimmung der Siebkoeffizienten für verschiedene Macromoleküle gemessen. Der Siebkoeffizient ist definiert als Quotient aus der Konzentration der Makromoleküle in der ursprünglichen Testlösung durch die Konzentration der Makromoleküle in der durch die Membran hindurchtretenden Lösung.

Man erhielt folgende Werte für den Siebkoeffizienten:

| Makromolekül | Molekulargewicht | Siebkoeffizient |
|---|---|---|
| Vitamin B12 | 1 300 | 0,87 |
| Inulin | 5 500 | 0,79 |
| Cytochrom C | 12 000 | 0.54 |
| a-Amylase | 45 000 | 0,24 |
| Bovin Serum Albumin | 60 000 | 0,16 |

Das bedeutet, daß Moleküle mit einem Molekulargewicht von 45000, das entspricht einem Moleküldurchmesser von 0,03 »m, von der Membran nahezu vollständig zurückgehalten werden.

### Beispiel 4:

### Vergleichsbeispiel: Herstellung einer PVDF-Flachmembran.

In einem Glaskolben mit Rührer wurden 27,5 Teile PVDF der Type Kynar 460 (Pennwalt), 18,85 Teile Triacetin, 4,71 Teile ε-Caprolactam und 48,94 Teile Dioctyladipat zusammengegeben. Unter Rühren wurde die Mischung auf 240°C aufgeheizt, bis man eine homogene Lösung erhielt. Danach wurde die Lösung auf 190°C abgekühlt. Diese Lösung wurde auf einer Gießwalze, die auf 20°C temperiert war, mit Hilfe eines Gießkastens zu einer Folie ausgeformt und auf einer Walze aufgewickelt.

Proben der Folien wurden 3 mal für 0,5 h in Isopropanol bei 60°C extrahiert, um die Lösungsmittel weitestgehend aus der Membran auszuwaschen. Nach dem Trocknen erhielt man eine weiße Flachmembran.

Die Membrandicke beträgt 140 »m, die maximale Porengröße der Membran 0,59 »m. Der Isopropanolfluß der Membran beträgt 9,4 ml/(cm² min bar).

Bei einem Transmembrandruck von 0,5 bar findet kein Wasserfluß durch die Membran statt.

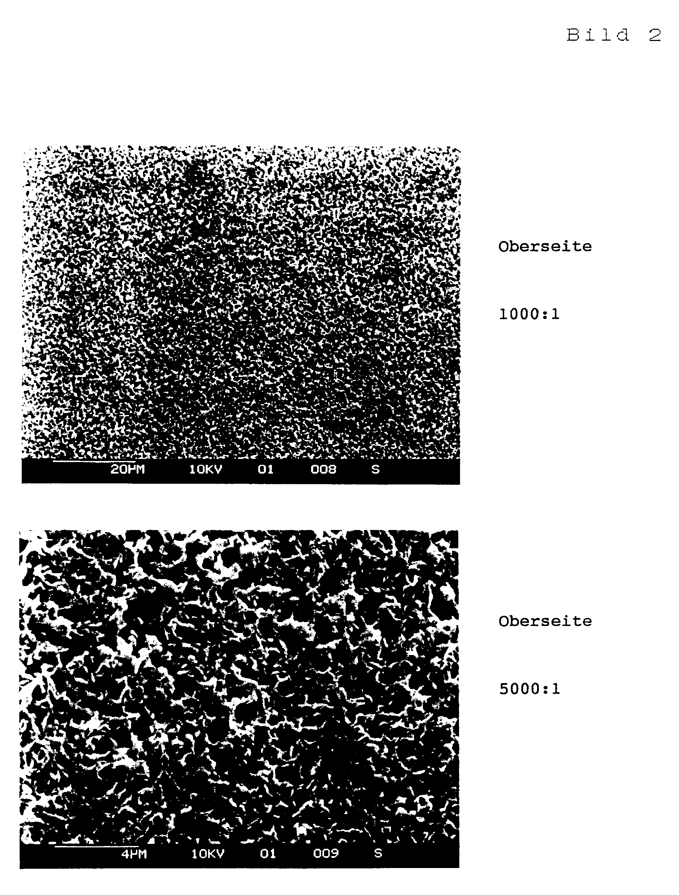

Die Oberflächen der Membran sind weniger offenporig als bei Beispiel 1 (vgl. Mikrofotografie, Bild 2).

### Beispiel 5:

### Herstellung einer PVDF/PEA-Flachmembran.

In einem Glaskolben mit Rührer wurden 56 g PVDF der Type Kynar 460 (Pennwalt), 132 g Dioctyladipat, 12 g Triacetin und 75 g einer 20 gewichtsprozentigen Lösung von Polyethylacrylat (A₅) in Triacetin zusammengegeben. Unter Rühren wurde die Mischung auf 240°C aufgeheizt, bis man eine homogene Lösung erhielt. Diese Lösung wurde auf einer Glasplatte zu einer Folie von 200 »m Schichtdicke ausgerakelt. Durch Eintauchen in Wasser wurde die Folie zum Erstarren gebracht. Die Folie wurde 3 mal für 0,5 h in Isopropanol bei 60°C extrahiert, um die Lösungsmittel weitestgehend aus der Membran auszuwaschen. Nach dem Trocknen erhielt man eine weiße Flachmembran. Der maximale Porendurchmesser betrug 0,82 »m, der Isopropanolfluß 9,1 ml/(cm² · min · bar).

### Beispiel 6:

### Herstellung einer PVDF/PMA/PEA-Flachmembran

In einem Glaskolben mit Rührer wurden 135 g PVDF der Type Kynar 460 (Pennwalt), 303,8 g Dioctyladipat, 29,2 g ε-Caprolactam, 117 g Triacetin und 10 g Polymethyl/ethylacrylat (A₆) zusammengegeben. Unter Rühren wurde die Mischung auf 240°C aufgeheizt, bis man eine homogene Lösung erhielt. Diese Lösung wurde auf 195°C abgekühlt und auf einer Glasplatte zu einer Folie von 200 »m Schichtdicke ausgerakelt. Durch Eintauchen in Wasser wurde die Folie zum Erstarren gebracht. Die Folie wurde 3 mal für 0,5 h in Isopropanol bei 60°C extrahiert, um die Lösungsmittel weitestgehend aus der Membran auszuwaschen. Nach dem Trocknen erhielt man eine weiße Flachmembran. Der maximale Porendurchmesser betrug 0,86 »m, der Isopropanolfluß 9,4 ml/(cm² · min · bar).

### Beispiel 7:

### Herstellung einer PVDF/PMA-Flachmembran mit DMSO als Lösungsmittel.

In einem Glaskolben mit Rührer wurden 54 g PVDF der Type Kynar 460 (Pennwalt) und eine Lösung von 6 g Polymethylacrylat (A₃) in 340 g Dimethylsulfoxid (DMSO) zusammengegeben. Unter Rühren wurde die Mischung auf 80°C aufgeheizt, bis man eine homogene Lösung erhielt. Der Lösung wurden 100 g Propylencarbonat zugegeben. Diese Lösung wurde auf Raumtemperatur abgekühlt, ohne daß eine Phasentrennung stattfand. Auf einer Glasplatte wurde die Lösung zu einer Folie von 200 »m Schichtdicke ausgerakelt. Die Folie wurde zuerst eine Minute an Luft gehalten und dann durch Eintauchen in Wasser zum Erstarren gebracht.

Die Folie wurde 3 mal für 0,5 h in Isopropanol bei 60°C extrahiert, um die Lösungsmittel weitestgehend aus der Membran auszuwaschen. Nach dem Trocknen erhielt man eine weiße Flachmembran.

Die maximale Porengröße der Membran beträgt 2,46 »m, der Isopropanolfluß der Membran 11,2 ml/(cm² min bar).

Die Membran wurde entsprechend Beispiel 16 mit Diglycerin und 2% Schwefelsäure behandelt. Die Eindringzeit (siehe Beispiel 10) der so behandelten Membran betrug 12 Sekunden.

### Beispiel 8:

### Herstellung von PVDF/PMA-Kapillarmembranen

Eine wie in Beispiel 1 hergestellte Lösung von 31,1 Teilen PVDF, 2,9 Teilen PMA (A₄), 22,44 Teilen Triacetin, 4,29 Teilen ε-Caprolactam und 44,55 Teilen Dioctyladipat wurde bei einer Temperatur von 210°C durch eine Ringspaltdüse zu einer Kapillarmembran versponnen. Zur Offenhaltung des Kapillarhohlraumes wurde durch eine Hohlnadel im Innern der Ringspaltdüse eine Mischung aus gleichen Teilen Rizinusöl und Dioctyladipat gefördert.

Die Membran wurde 3 mal eine halbe Stunde in Isopropanol bei 60°C extrahiert. Die extrahierte Membran hat eine maximale Porengröße von 0,63 »m und einen Isopropanolfluß von 9,24 ml/(cm² min bar). Die Kapillarmembran hat einen Innendurchmesser von 1,0 mm und einen Außendurchmesser von 1,5 mm.

Die Membran ist mit Wasser nicht benetzbar.

### Beispiel 9:

### Herstellung einer PVDF Kapillarmembran

Entsprechend Beispiel 8 wurde eine PVDF-Lösung, bestehend aus 29,9 Teilen PVDF, 18,2 Teilen Triacetin, 4,6 Teilen ε-Caprolactam und 47,3 Teilen Dioctyladipat ohne den Zusatz eines Polyacrylates zu einer Kapillarmembran gesponnen.

Die Membran wurde 3 mal eine halbe Stunde in Isopropanol bei 60°C extrahiert.

Die extrahierte Membran hat eine maximale Porengröße von 0,54 »m und einen Isopropanolfluß von 6,55 ml/(cm² min bar).
Die Membran ist mit Wasser nicht benetzbar. Die Kapillarmembran hat einen Innendurchmesser von 1,0 mm und einen Außendurchmesser von 1,5 mm.

### Beispiel 10:

### Umsetzung von PVDF/PEA mit H₂SO₄

Proben der Membran nach Beispiel 5 wurden mit Schwefelsäure umgesetzt.

Dazu wurden die Membranen erst mit Ethanol benetzt. Die Membranen wurden dann durch Austauschen des Alkohols gegen demineralisiertes Wasser, dann gegen 70 prozentige Schwefelsäure und dann gegen konzentrierte Schwefelsäure (98%) ohne Einlagerung von Gasblasen in der Membranstruktur mit der konzentrierten Schwefelsäure in Kontakt gebracht. Die Membranen verblieben für 1,5 h bei 60°C in der Schwefelsäure.

Danach wurden die Membranen erst mit 70 prozentiger Schwefelsäure und dann mit demineralisiertem Wasser ausgewaschen und anschließend getrocknet. Die trockenen Proben waren hydrophil.

Die Eindringzeit betrug ca. 30 Sekunden.

Unter Eindringzeit wird in diesem und in den folgenden Beispielen die Zeit verstanden, nach der ein Wassertropfen von 2 »l, der mit einer Präzisionspipette auf die Membran aufgegeben wird, vollständig verschwunden ist. Als Vergleich dient die Zeit von mehr als etwa 300 Sekunden, die der Wassertropfen benötigt, um auf einem Glasstab durch Verdunsten vollständig zu verschwinden. Bei einer hydrophilen Membran dauert es naturgemäß weniger lang, bis der Wassertropfen verschwunden ist, da er von der Membranstruktur aufgesogen wird. Wegen der nicht immer gleichen Membranstruktur, welche auch die Eindringzeiten beeinflußt, bietet die Bestimmung der Eindringzeit nur ein halbquantitatives, aber ausreichendes und vor allem praxisnahes Maß für die Hydrophilie einer Membran, da es bei der praktischen Anwendung der Membran auf ein vollständiges Benetzen der Membran mit Wasser innerhalb einer bestimmten Zeit ankommt.

### Beispiel 11:

### Umsetzung von PVDF/PMA mit Borax-Lösung

Eine Probe der Flachmembran nach Beispiel 5 wurde mit Isopropanol benetzt und danach in 5 gewichtsprozentiger Natronlauge, die mit Borax gesättigt war, im Druckgefäß 8 Stunden bei 120°C behandelt. Anschließend wurde die Membran mit demineralisiertem Wasser ausgespült und getrocknet. Die Membran war leicht bräunlich und hydrophil. Die Eindringzeit betrug etwa 40 Sekunden.

### Beispiel 12:

### Umsetzung mit Glycerin und Diglycerin

Kapillarmembranen, die analog den Beispielen 8 und 9 hergestellt waren und einen unterschiedlichen Gehalt an PMA, bezogen auf das Polymer, aufwiesen, wurden mit Glycerin bzw. Diglycerin als Hydrophilierungslösung behandelt. Die Behandlungszeit betrug jeweils 6 Stunden bei 140°C. Der Hydrophilierungslösung war 1 Gew.-% Perchlorsäure zugesetzt worden.

Die Membranproben wurden dabei zuerst mit Ethanol benetzt und dann in die jeweilige Hydrophilierungslösung eingetaucht. Nach der Behandlung wurden die Membranen mit demineralisiertem Wasser gewaschen und getrocknet. An den trockenen Membranmustern wurden die Eindringzeiten bestimmt.

| Membran: PVDF und | Glycerin | Diglycerin | Diglycerin + 10% Wasser |
|---|---|---|---|
| | Eindringzeiten in Sekunden | | |
| 0 % PMA | > 300 | > 300 | > 300 |
| 13,5 % PMA | 1 - 2 | 1 | 1 |
| 8,5 % PMA | 2 - 3 | 2 - 3 | 1 - 2 |

### Beispiel 13:

### Umesterung von PVDF/PMA/PEA mit Diglycerin

Eine Membran nach Beispiel 6 wurde nach Vorbenetzung mit Ethanol in eine Lösung aus Diglycerin und 6 Gew.-% konzentrierter Schwefelsäure (98 %) gegeben. Die Lösung wurde für 8 Stunden auf 140°C gehalten. Anschließend wurden die Membranen herausgenommen, mehrfach mit demineralisiertem Wasser extrahiert und getrocknet.

Die Eindringzeit an der trockenen Membran betrug 2 - 3 Sekunden.

### Beispiel 14:

### Umesterung von PVDF mit 2 % PMA

Eine Flachmembran, die analog Beispiel 5 aus PVDF und 2 % PMA hergestellt worden war, wurde wie in Beispiel 13 mit Diglycerin und 2 Gew.-% konzentrierter Schwefelsäure 20 Stunden bei 140°C behandelt. Nach dem Auswaschen und Trocknen der Membran betrug die Eindringzeit 70 Sekunden.

### Beispiel 15:

### Umesterung von PVDF/PMA mit verschiedenen Alkoholen

Membranproben, die nach Beispiel 8 hergestellt worden waren, wurden mit Methanol benetzt und mit verschiedenen Alkoholen bei 140°C 6 Stunden unter Zugabe von 1 Gew.-% Perchlorsäure umgesetzt.

| Alkohol: | Eindringzeit (s) |
|---|---|
| Glycerin | 3 |
| Diglycerin | 1 |
| Polyglycerin | 2 |
| Ethylenglycol | > 300 |
| Polyethylenglycol 300 | > 300 |

| 10 Gew.-% Alkohol in Ethylenglycol: | |
|---|---|
| Sorbit | 2 |
| Saccarose | 2 |
| Stärke | > 300 |
| Pentaerythrit | 3 |
| TRIS | 60 |
| (TRIS = Tris(hydroxymethyl)aminomethan) | |

### Beispiel 16:

### Umesterung von PVDF/PMA-Flachmembranen mit Diglycerin und verschiedenen Säuren

Membranproben nach Beispiel 1 wurden mit einer Lösung aus Diglycerin und jeweils 4 verschiedenen konzentrierten Säuren umgesetzt. Die Behandlung dauerte 8 Stunden bei 140°C. Es sind die Eindringzeiten der gespülten und getrockneten Membranen angegeben.

| Säure | Eindringzeit (s) |
|---|---|
| Salzsäure (35%-ig) | 8 |
| Perchlorsäure (70%-ig) | 2 |
| Schwefelsäure (98%-ig) | 2 |
| Phosphorsäure (85%-ig) | > 300 |

Im Gegensatz zu den Membranen aus Beispiel 1 und 4 weisen die hydrophilen Membranproben nach Beispiel 16 einen Wasserfluß von etwa 7,4 ml/(cm² min bar) bei 0,5 bar Transmembrandruck auf.

### Beispiel 17:

### Bestimmung der Hydroxylgruppen

An der mit Diglycerin/Schwefelsäure hydrophilierten Membran aus Beispiel 16 wurde die Hydroxylzahl als Maß für die Menge freier OH-Gruppen an der Membranoberfläche bestimmt.

Die Membran wurde dazu mit einem Acetylierungsgemisch umgesetzt. Die verbrauchte Menge wird durch Rücktitration der Lösung bestimmt.

### Herstellen des Acetylierungsgemisches:

3,5 ml Perchlorsäure (70 %) werden mit 150 ml Essigsäureethylester und 5 ml Essigsäureanhydrid zusammengegeben. Zu 4 Teilen der Lösung wird ein Teil Hexan zugegeben.

### Messung:

Ein abgewogenes Membranstück wurde in einen Erlenmayerkolben gegeben, dazu wurden 5 ml Acetylierungsgemisch und 2 ml Wasser gegeben. Nach 5 Minuten wurden 25 ml einer Mischung aus 3 Teilen Pyridin und 1 Teil Wasser zugegeben. Nach weiteren 5 Minuten wurde mit 50 ml Isopropanol verdünnt und mit Phenolphthalein als Indikator mit 1 normaler Natronlauge titriert.

Der Gehalt an OH-Gruppen wurde zu 0,3 mVal/g bestimmt.

### Beispiel 18:

### Herstellung einer hydrophilen Vergleichsmembran gemäß der DE-A-27 35 887

Analog zu Beispiel 29 der Patentschrift DE 27 35 887 wurde eine hydrophobe Kapillarmembran aus PVDF (nach Beispiel 9) in eine wässrige Lösung mit 6 Gew.-% Polyvinylalkohol getaucht. Die Membran wurde getrocknet und anschließend 20 Minuten bei 90°C und 15 Minuten bei 140°C behandelt. Die Membran wurde 2 mal 10 Minuten mit Wasser bei 90°C gespült. Die getrocknete Membran wurde bei Raumtemperatur für zwei Minuten in ein Acetalisierungsbad aus 20 Teilen Schwefelsäure (96%), 2 Teilen Natriumsulfat und 100 Teilen einer 40 gewichtsprozentigen Formaldehydlösung in Wasser getaucht. Die Membranen wurden danach mit Wasser gespült und getrocknet.

An der getrockneten Membran wurde eine Eindringzeit von 59 Sekunden gemessen.

### Beispiel 19:

### Behandlung von hydrophilen Membranen mit Hypochloritlösung

Eine Kapillarmembran nach Beispiel 12 mit 8,5 Gew.% PMA und Glycerinbehandlung und die Vergleichsmembran nach Beispiel 16 wurden 16 Stunden in einer wässrigen Lösung mit 5000 ppm Natriumhypochlorit gelagert, anschließend mit Wasser gespült und getrocknet. Die erfindungsgemäße Membran war noch gut hydrophil, während die Vergleichsmembran nicht mehr von Wasser benetzt wurde, wie aus dem Vergleich der Eindringzeiten zu erkennen ist.

| Membran | Eindringzeit (s) |
|---|---|
| nach Beispiel 10 | 15 |
| nach Beispiel 16 | > 300 |

### Beispiel 20:

### Vergleich mit einer hydrophilen PVDF-Membran der Firma Millipore

Eine kommerziell erhältliche hydrophile Flachmembran aus PVDF von Millipore und eine erfindungsgemäße PVDF Flachmembran, die nach Beispiel 16 durch Behandlung mit Schwefelsäure und Diglycerin hydrophiliert wurde, wurden 16 Stunden in konzentrierter Schwefelsäure aufbewahrt. Anschließend wurden die Membranen mit Wasser gespült und getrocknet.

Die erfindungsgemäße Membran war noch gut hydrophil, während die Vergleichsmembran nicht mehr von Wasser benetzt wurde, wie aus dem Vergleich der Eindringzeiten zu erkennen ist.

| Membran | Eindringzeit (s) |
|---|---|
| Nach Beispiel 16 | 12 |
| Millipore | > 300 |

### Beispiel 21

### Behandlung einer PVDF/PMA-Kapillarmembran gemäß Beispiel 8 mit Tetraethylenpentamin

Es wurde eine Reaktionslösung hergestellt, indem eine wässrige Lösung mit 40 Gew.-% Tetraethylenpentamin bei 80°C mit Ammoniumchlorid gesättigt wurde. Eine Kapillarmembran nach Beispiel 8 wurde mit Ethanol benetzt und dann bei 80°C in die Lösung getaucht. Nach dem Abdampfen des Ethanols wurde die Lösung mit der Membran in einem Druckgefäß auf 140°C aufgeheizt. Nach verschiedenen Zeiten wurden Membranproben aus der Lösung herausgenommen, mit Wasser gewaschen und getrocknet. Die Membranen waren nur geringfügig verfärbt und nach einer Reaktionszeit von mehr als 3 Stunden gut mit Wasser benetzbar.

| Reaktionszeit (h) | Eindringzeit (s) |
|---|---|
| 1 | > 300 |
| 3 | > 300 |
| 5 | 26 |
| 7 | 2 |
| 16 | 2 |

Eine in gleicher Weise behandelte Vergleichsmembran nach Beispiel 9 ohne Zusatz von Polyacrylat zeigte auch nach 16 Stunden Reaktionszeit keine Hydrophilie.

### Beispiel 22:

### Behandlung einer PVDF/PMA-Kapillarmembran gemäß Beispiel 8 mit Glutaminsäure

Es wurde eine Reaktionslösung hergestellt, indem eine wässrige Lösung von 40 Gew.-% Glutaminsäure mit Ammoniak auf pH 10 gepuffert wurde. Eine Kapillarmembran nach Beispiel 8 wurde mit Ethanol benetzt und dann bei 80°C in die Lösung getaucht. Nach dem Abdampfen des Ethanols wurde die Lösung mit der Membran in einem Druckgefäß auf 140°C aufgeheizt. Nach 16 Stunden wurde die Membran aus der Lösung herausgenommen, mit Wasser gewaschen und getrocknet.

Die Membran hatte eine Eindringzeit von 18 Sekunden. Eine Vergleichsmembran nach Beispiel 9, die auf die gleiche Weise behandelt wurde, war nicht mit Wasser benetzbar und zeigte somit eine Eindringzeit > 300 Sekunden.

### Beispiel 23:

### Beständigkeit gegen Natronlauge

Je eine Flachmembran nach Beispiel 1 und Beispiel 4, sowie eine hydrophile, mit Diglycerin umgeesterte Flachmembran nach Beispiel 15 wurden mit Methanol benetzt und in 10 gewichtsprozentige Natronlauge bei 40°C gelegt. Es wurde die Zeit bis zur ersten Verfärbung der Membranproben gemessen.

Eine Verfärbung der Membran zeigt eine chemische Veränderung der Membran durch den Angriff von Natronlauge an.

| Membran | Zeit bis zur ersten Verfärbung |
|---|---|
| Beispiel 1 | 50 Minuten |
| Beispiel 4 hydrophile | 5 Minuten |
| Membran nach Beispiel 15 | 45 Minuten |

### Beispiel 24

### Affinitätschromatographie mit einem chemisch stabilen Liganden

a) Membran mit Cibacron-Blau
Hohlfasermembrandaten:

| | |
|---|---|
| Innerer Durchmesser: | 960 - 1120 »m; |
| Wand: | 230 »m; |
| PVDF: | 90,5 Gew.-%; |
| PMA: | 9,5 Gew.-% |
| Gehalt an OH-Gruppen: | 0,012 m Val/g Membran, erhalten nach Beispiel 12 durch Umsetzung einer 8,5% PMA enthaltenden Kapillarmembran mit Glycerin |
| Maximaler Porendurchmesser: | 0,79 »m; |
| Transmembranfluß von Isopropanol: | 19,4 ml/min·cm²·bar. |

Moduldaten: effektive Länge 155 mm; 62 Fasern (5,6 g; 314 cm² Innenwandfläche) Vorbehandlung: Die Aktivierung der Membran erfolgt durch Umpumpen der entsprechenden Lösungen durch die Membran. Die Membran wird mit 300 mg Cibacron Blue 3GA in 60 ml Wasser 45 Minuten behandelt. Danach wird bis zur Sättigung festes NaCl zugegeben und weitere 30 Minuten behandelt. Anschließend wird auf 80°C erhitzt, 600 mg Na₂CO₃ zugegeben und weitere 2 Stunden bei dieser Temperatur belassen. Nach dem Abkühlen wird mit 2 l Wasser im single-pass gewaschen.
b) Chromatographie
I. Reinigung von Hexokinase
Eine rohe Präparation von Hexokinase (From Yeast, Sigma Best.- Nr. H 5125) wird in 50 ml 5 mM (Millimolar) TRIS-HCl-Puffer p_{H} 6,4 gelöst und durch die Membran filtriert. Zum Waschen werden 2 x 50 ml desselben Puffers benutzt. Eluiert wird mit 50 ml 20 mM TRIS-HCl-Puffer p_{H} 8,6. Die Ergebnisse sind in Tabelle 1 aufgeführt.

**Tabelle 1**

| Hexokinase: | Aktivität [IU]* | Protein [mg] | spezifische Aktivität [IU/mg] |
|---|---|---|---|
| Ausgangslösung: | 528 | 22.55 | 23.41 |
| Restlösung: | 394 | 17.14 | 22.99 |
| Spüllösung: | 9 | 0.39 | 23.08 |
| gebunden | 125(100%) | 5.02 | |
| eluiert: | 122( 97,6%) | 2.77 | 44.04 |

| | | | |
|---|---|---|---|
| *) International Units (1 Mikromol Umsatz/min unter Standardbedingungen) | | | |

Wie aus der Tabelle 1 hervorgeht, wurde nahezu alle adsorbierte Hexokinase-Aktivität auch wieder eluiert, wobei die spezifische Aktivität der Hexokinase nahezu verdoppelt wurde. Die Hexokinase ist bei dieser Reinigungsstufe naturgemäß noch nicht zur Homogenität gereinigt.
II. Adsorption von Albumin
Human-Albumin (Serva, Best.-Nr. 11870) wird, in 50 ml 100 mM KCl in 50 mM TRIS-HCl-Puffer p_{H} 7,0 gelöst, durch Umpumpen an der Membran adsorbiert. Nach dem Waschen des Moduls mit demselben Puffer ( 2 x 50 ml) wird mit 50 ml 1,5 M KCl-Lösung in 50 mM Phosphatpuffer p_{H} 7,0 desorbiert. Die Ergebnisse sind in Tabelle 2 zusammengefaßt.

**Tabelle 2**

| Albumin aus Humanserum | Protein [mg] |
|---|---|
| Ausgangslösung (50 ml) | 22.10 |
| Restlösung (50 ml) | 4.45 |
| Spüllösung 1 (50 ml) | 6.06 |
| Spüllösung 2 (50 ml) | 1.11 |
| gebunden | 10.48 |
| eluiert | 10.43 |

Wie aus der Tabelle 2 ersichtlich ist, wird alles adsorbierte Albumin auch wieder desorbiert.
c) Regenerierung des Moduls
Die Reinigung kann zwar analog Blue Sepharose (z.B. von der Firma Pharmacia) mit 0,1 M TRIS-Puffer p_{H} 9,3 und Acetat-Puffer 3,2 bei verminderter Effektivität und längerer Zeitdauer durchgeführt werden. Wesentlich effektiver ist jedoch die Reinigung mit 0,1 N NaOH oder 1 N HCl, die überraschenderweise von der derivatisierten Membran ohne Schädigung ausgehalten wird (p_{H} = 1 und 13). Bei der Reinigung mit Säure erfolgt ein reversibler Farbumschlag von blau nach rot, der auf eine Protonierung des aromatischen Systems von Cibacronblau zurückzuführen ist. Durch die sehr stabile Bindung von Matrix und Farbstoff wird unter diesen Bedingungen keine meßbare Menge Farbstoff abgespalten. Der so gereinigte Modul entspricht dem Neuzustand.
Allgemeine Anmerkungen: Die Proteingehalte werden nach Biuret getestet. Die Hexokinase-Aktivität wird nach: H.U. Bergmeyer, Methoden der enzymatischen Analyse, 3. Auflage (1974), Verlag Chemie, Weinheim, Seiten 502 - 503, bestimmt.

### Beispiel 25

### Wiederbeladung mit kovalent immobilisiertem Enzym

Die Hydrophilierung der Flachmembran erfolgt analog wie in Beispiel 21 beschrieben. Anschließend erfolgt die Umsetzung der Membran (effektive Fläche 32 cm², effektives Gewicht 0,31 g, 0,014 mVal NH₂-Gruppen/g Membran) mit 10%-iger Glutardialdehyd-Lösung in 10 mM Phosphat-Puffer pH 7,0 über 1 Stunde bei Raumtemperatur. Die Invertase-Lösung (E.C.3.2.1.26; from yeast, Fa. Boehringer Mannheim, Kat.-Nr. 104922, spez. Aktivität 330 U/mg) in 10 mM Acetatpuffer pH 4,6 wird im Überschuß über Nacht im Kreislauf durch die aktivierte Membran filtriert. Unter diesen Bedingungen bleibt die katalytische Aktivität der immobilisierten Invertase nahezu vollständig erhalten.

Um die Membran z.B. nach einer Proteindenaturierung erneut mit Protein zu beladen, wird die Membran nochmals der Aminolyse aus Beispiel 21 unterworfen. Die zweite Beladung der Membran mit Invertase erfolgt unter denselben Bedingungen wie oben.

Bestimmung der immobilisierten Invertase:

| 1.Immobilisierung: | |
|---|---|
| Ausgangslösung: | 3060 IU |
| Restlösung, nicht immobilisiert: | 1680 IU |
| Immobilisierte Invertase: | 1380 IU |

| 2.Immobilisierung: | |
|---|---|
| Ausgangslösung: | 2510 IU |
| Restlösung, nicht immobilisiert: | 1180 IU |
| Immobilisierte Invertase: | 1330 IU |

## Patentansprüche

1. Flach- oder Kapillarmembran auf der Basis eines homogenen Gemisches aus Polyvinylidenfluorid und eines zweiten, durch chemische Umsetzung hydrophilierbaren Polymeren, dadurch gekennzeichnet, daß sie aus einem homogenen Gemisch von 70 bis 98 Gewichtsprozent Polyvinylidenfluorid und 2 bis 30 Gewichtsprozent aus einem im wesentlichen aus Polyacrylsäuremethyl- und/oder -ethylester gebildeten Polymeren besteht und eine maximale Porengröße im Bereich von 0,005 bis 10 »m aufweist.

2. Flach- oder Kapillarmembran nach Anspruch 1, dadurch gekennzeichnet, daß sie eine maximale Porengröße im Bereich von 0,01 bis 2 »m aufweist.

3. Flach- oder Kapillarmembran nach Anspruch 2, dadurch gekennzeichnet, daß sie eine maximale Porengröße von 0,05 bis 0,8 »m aufweist.

4. Flach- oder Kapillarmembran nach einem oder mehreren der Ansprüche 1 bis 3, dadurch gekennzeichnet, daß das Polyvinylidenfluorid ein mittleres Molekulargewicht (Gewichtsmittel) von 30 000 bis 500 000 und das im wesentlichen aus Polyacrylsäuremethyl- und/oder -ethylester gebildete Polymer ein mittleres Molekulargewicht von 5 000 bis 1 000 000 besitzt.

5. Flach- oder Kapillarmembran nach Anspruch 4 dadurch gekennzeichnet, daß das Polyvinylidenfluorid ein mittleres Molekulargewicht von 50 000 bis 500 000 und das im wesentlichen aus Polyacrylsäuremethyl- und/oder -ethylester gebildete Polymer ein mittleres Molekulargewicht von 50 000 bis 200 000 besitzt.

6. Flach- oder Kapillarmembran nach einem oder mehreren der Ansprüche 1 bis 5, dadurch gekennzeichnet, daß sie aus einem homogenen Gemisch von 80 bis 95 Gew.-% Polyvinylidenfluorid und 5 bis 20 Gew.-% aus einem im wesentlichen aus Polyacrylsäuremethyl- und/oder -ethylester gebildeten Polymeren besteht.

7. Flach- oder Kapillarmembran nach einem oder mehreren der Ansprüche 1 bis 6, dadurch gekennzeichnet, daß sie auf ihrer gesamten Oberfläche 0,001 bis 10 mVal/g Membran -OH, -NH₂ oder -COOH-Gruppen oder Gemische dieser hydrophilen funktionellen Gruppen enthält.

8. Flach- oder Kapillarmembran nach Anspruch 7, dadurch gekennzeichnet, daß sie auf ihrer gesamten Oberfläche 0,01 bis 5 mVal/g Membran -OH, -NH₂ oder -COOH-Gruppen oder Gemische dieser hydrophilen funktionellen Gruppen enthält.

9. Verfahren zur Herstellung der Flach- oder Kapillarmembran nach einem oder mehreren der Ansprüche 1 bis 6, dadurch gekennzeichnet, daß man, bezogen auf das Polymergesamtgewicht, aus 70 bis 98 Gewichtsprozent Polyvinylidenfluorid und 2 bis 30 Gewichtsprozent eines im wesentlichen aus Polyacrylsäuremethyl- und/oder -ethylester gebildeten Polymeren unter Verwendung von einem oder mehreren Lösungsmitteln und einem oder mehreren Nichtlösern eine 10 bis 40 Gew.-%-ige Lösung, bezogen auf deren Gesamtgewicht, herstellt, die oberhalb Raumtemperatur im flüssigen Zustand einen Bereich völliger Mischbarkeit und eine Mischungslücke aufweist und oberhalb Raumtemperatur einen Erstarrungsbereich besitzt, indem man die Stoffkomponenten unter intensivem homogenen Mischen auf eine Temperatur oberhalb der Mischungslücke aufheizt, die so erhaltene Lösung von der Temperatur oberhalb der Mischungslücke in einer Abkühlflüssigkeit schnell abkühlt und gleichzeitig zu einer Flach- oder Kapillarmembran ausformt und anschließend die Membran durch Extraktion von Lösungsmittel -und Nichtlöserresten befreit.

10. Verfahren nach Anspruch 9, dadurch gekennzeichnet, daß eine 20 bis 35 Gew.-%ige Lösung hergestellt wird.

11. Verfahren nach einem oder zwei der Ansprüche 9 bis 10, dadurch gekennzeichnet, daß eine Lösung mit einer Temperatur von 160 bis 230°C hergestellt wird.

12. Verfahren nach einem oder mehreren der Ansprüche 9 bis 11, dadurch gekennzeichnet, daß eine Lösung hergestellt wird, die, bezogen auf das Polymergesamtgewicht, 80 bis 95 Gewichtsprozent Polyvinylidenfluorid und 5 bis 20 Gewichtsprozent Polyacrylsäuremethyl- und/oder -ethylester enthält.

13. Verfahren nach einem oder mehreren der Ansprüche 9 bis 12, dadurch gekennzeichnet, daß zur Herstellung der Lösung ein Polyvinylidenfluorid eines mittleren Molekulargewichts von 30 000 bis 500 000 und ein im wesentlichen aus Polyacrylsäuremethyl- und/oder -ethylester gebildetes Polymer eines mittleren Molekulargewichts von 50 000 bis 1 000 000 verwendet wird.

14. Verfahren nach Anspruch 13, dadurch gekennzeichnet, daß ein Polyvinylidenfluorid eines mittleren Molekulargewichts von 50 000 bis 500 000 und ein im wesentlichen aus Polyacrylsäuremethyl- und/oder -ethylester gebildetes Polymer eines mittleren Molekulargewichts von 50 000 bis 200 000 verwendet wird.

15. Verfahren nach einem oder mehreren der Ansprüche 9 bis 14, dadurch gekennzeichnet, daß bei der Herstellung der Lösung als Lösungsmittel eine oder mehrere Verbindungen aus der Gruppe von Glycerintriacetat, Glycerindiacetat, 2-(2-Butoxyäthoxy-)ethylacetat und ε-Caprolactam verwendet wird bzw. werden.

16. Verfahren nach Anspruch 15, dadurch gekennzeichnet, daß als Lösungsmittel Glycerintriacetat oder ein Gemisch aus Glycerintriacetat und ε-Caprolactam verwendet wird.

17. Verfahren nach einem oder mehreren der Ansprüche 9 bis 16, dadurch gekennzeichnet, daß als Nichtlöser Di-n-octyladipat oder Rizinus-Öl oder ein Gemisch hiervon verwendet wird.

18. Verfahren nach einem oder mehreren der Ansprüche 9 bis 17, dadurch gekennzeichnet, daß als Abkühlungsflüssigkeit Wasser, gegebenenfalls mit einem Zusatz an Tensid, verwendet wird.

19. Verfahren nach einem oder mehreren der Ansprüche 9 bis 18, dadurch gekennzeichnet, daß zur Extraktion der Membran Isopropanol verwendet wird.

20. Verfahren zur Herstellung der Flach- oder Kapillarmembran nach einem oder mehreren der Ansprüche 1 bis 6, dadurch gekennzeichnet, daß man, bezogen auf das Polymergesamtgewicht, aus 70 bis 98 Gewichtsprozent Polyvinylidenfluorid und 2 bis 30 Gewichtsprozent eines im wesentlichen aus Polyacrylsäuremethyl- und/oder -ethylester gebildeten Polymeren unter Verwendung von einem oder mehreren aprotischen Lösungsmitteln eine 10 bis 40 Gew.-%ige Lösung, bezogen auf deren Gesamtgewicht, herstellt, die Lösung zu einer Flach- oder Kapillarmembran, letztere gegebenenfalls unter Zuhilfenahme einer Innenflüssigkeit, ausformt und durch Koagulation in einem Nichtlöserbad in die feste Phase überführt und anschließend die Membran durch Extraktion von Lösungsmittelresten befreit.

21. Verfahren nach Anspruch 20, dadurch gekennzeichnet, daß bei der Herstellung der Lösung als aprotonisches Lösungsmittel eine oder mehrere Verbindungen aus der Gruppe von N-Methylpyrrolidon, Dimethylsulfoxid, Dioxan, Dimethylformamid und Dimethylacetamid verwendet wird bzw. werden.

22. Verfahren nach einem oder zwei der Ansprüche 20 bis 21, dadurch gekennzeichnet, daß die Temperatur der Lösung und die Temperatur des Nichtlöserbades 0 bis 80°C beträgt.

23. Verfahren nach Anspruch 22, dadurch gekennzeichnet, daß die Temperatur der Lösung und die Temperatur des Nichtlöserbades 20 bis 50°C beträgt.

24. Verfahren nach einem oder mehreren der Ansprüche 20 bis 23, dadurch gekennzeichnet, daß zur Koagulation der Membran als Nichtlöser ein Alkohol mit 1 bis 12 C-Atomen oder Wasser oder Gemische davon verwendet werden.

25. Verfahren nach einem oder mehreren der Ansprüche 20 bis 24, dadurch gekennzeichnet, daß zur Extraktion der Membran ein Alkohol mit 1 bis 3 C-Atomen verwendet wird.

26. Verfahren nach einem oder mehreren der Ansprüche 20 bis 25, dadurch gekennzeichnet, daß zur Herstellung der Lösung ein Polyvinylidenfluorid eines mittleren Molekulargewichts von 30 000 bis 500 000 und ein im wesentlichen aus Polyacrylsäuremethyl- und/oder -ethylester gebildetes Polymer eines mittleren Molekulargewichts von 5 000 bis 1 000 000 verwendet wird.

27. Verfahren nach Anspruch 26, dadurch gekennzeichnet, daß ein Polyvinylidenfluorid eines mittleren Molekulargewichts von 50 000 bis 500 000 und ein im wesentlichen aus Polyacrylsäuremethyl- und/oder -ethylester gebildetes Polymer eines mittleren Molekulargewichts von 50 000 bis 200 000 verwendet wird.

28. Verfahren zur Herstellung der Flach- oder Kapillarmembran nach einem oder zwei der Ansprüche 7 bis 8, dadurch gekennzeichnet, daß die Estergruppen auf der Gesamtoberfläche der Membran gemäß einem oder mehreren der Ansprüche 1 bis 6 einer zumindest teilweisen Hydrolyse und/oder einer zumindest teilweisen Umesterung mit einem mindestens dreiwertigen Alkohol mit 3 - 12 C-Atomen und/oder einer zumindest teilweisen Aminolyse mit einer Aminoverbindung mit 2 - 8 C-Atomen unterworfen werden.

29. Verfahren nach Anspruch 28, dadurch gekennzeichnet, daß man die Estergruppen auf der Gesamtoberfläche der Membran mit konzentrierter Schwefelsäure bei einer Temperatur von 40 bis 80°C 1 bis 20 Stunden lang einer zumindest teilweisen Hydrolyse unterwirft.

30. Verfahren nach Anspruch 28, dadurch gekennzeichnet, daß man die Estergruppen auf der Gesamtoberfläche der Membran mit bei Raumtemperatur gesättigter wäßriger Boraxlösung, die bis zu 5 Gew.-% Alkalilauge enthalten kann, bei einer Temperatur von 80 bis 140°C und einem p_{H}-Wert von 9 - 11 1 bis 20 Stunden lang einer zumindest teilweisen Hydrolyse unterwirft.

31. Verfahren nach Anspruch 28, dadurch gekennzeichnet, daß man die Estergruppen auf der Gesamtoberfläche der Membran mit einem mindestens dreiwertigen Alkohol mit 3 - 12 C-Atomen unter Zusatz von 0,1 bis 10 Gew.-%, bezogen auf den mehrwertigen Alkohol, einer starken Säure bei einer Temperatur von 100 bis 150°C 1 bis 30 Stunden lang einer zumindest teilweisen Umesterung unterwirft.

32. Verfahren nach Anspruch 31, dadurch gekennzeichnet, daß als mindestens dreiwertiger Alkohol eine oder mehrere Verbindungen aus der Gruppe von Glycerin, Diglycerin, Triglycerin, einem Polyglyceringemisch, Pentaerythrit und Sorbit verwendet wird bzw. werden.

33. Verfahren nach Anspruch 28, dadurch gekennzeichnet, daß man die Estergruppen auf der Gesamtoberfläche der Membran mit mindestens einer Aminoverbindung mit 2 bis 8 C-Atomen unter Verwendung von entsprechenden Puffergemischen bei einem p_{H}-Wert < 11 und einer Temperatur von 50 bis 150°C ein bis 24 Stunden lang einer zumindest teilweisen Aminolyse unterwirft.

34. Verfahren nach Anspruch 33, dadurch gekennzeichnet, daß als Aminoverbindung eine oder mehrere Verbindungen aus der Gruppe von Diäthylentriamin, Triäthylentetramin und Tetraäthylenpentamin verwendet wird bzw. werden.

35. Verwendung der Flach- oder Kapillarmembran nach einem oder zwei der Ansprüche 7 bis 8 zur Immobilisierung biochemisch aktiver Verbindungen.

## Claims

1. Flat or capillary membrane based on a homogeneous mixture of polyvinylidene fluoride and a second polymer, capable of being rendered hydrophilic by chemical conversion, characterised in that it consists of a homogeneous mixture of from 70 to 98% by weight of polyvinylidene fluoride and from 2 to 30% by weight of a polymer formed essentially from polymethyl acrylate or polyethyl acrylate and has a maximum pore size in the range of from 0.005 to 10 »m.

2. Flat or capillary membrane according to claim 1, characterised in that it has a maximum pore size in the range of from 0.01 to 2 »m.

3. Flat or capillary membrane according to claim 2, characterised in that it has a maximum pore size in the range of from 0.05 to 0.8 »m.

4. Flat or capillary membrane according to one or more of claims 1 to 3, characterised in that the polyvinylidene fluoride has an average molecular weight (weight average) of from 30,000 to 500,000 and the polymer formed essentially from polymethyl acrylate or polyethyl acrylate has an average molecular weight of from 5,000 to 1,000,000.

5. Flat or capillary membrane according to claim 4, characterised in that the polyvinylidene fluoride has an average molecular weight of from 50,000 to 500,000 and the polymer formed essentially from polymethyl acrylate or polyethyl acrylate has an average molecular weight of from 50,000 to 200,000.

6. Flat or capillary membrane according to one or more of claims 1 to 5, characterised in that it consists of a homogeneous mixture of from 80 to 95% by weight of polyvinylidene fluoride and from 5 to 20% by weight of a polymer formed essentially from polymethyl acrylate or polyethyl acrylate.

7. Flat or capillary membrane according to one or more of claims 1 to 6, characterised in that it contains on its total surface from 0.001 to 10 mVal/g membrane of -OH, -NH₂ or -COOH groups or mixtures of these hydrophilic functional groups.

8. Flat or capillary membrane according to claim 7, characterised in that it contains on its total surface from 0.01 to 5 mVal/g membrane of -OH, -NH₂ or -COOH groups or mixtures of these hydrophilic functional groups.

9. Method for the preparation of the flat or capillary membrane according to one or more of claims 1 to 6, characterised in that a 10 to 40% by weight solution, referred to the total weight thereof, is prepared from 70 to 98% by weight of polyvinylidene fluoride and from 2 to 30% by weight of a polymer formed essentially from polymethyl acrylate or polyethyl acrylate, referred to the total weight of polymer, using one or more solvents and one or more nonsolvents, which solution shows a range of complete miscibility and a miscibility gap in the liquid state above room temperature and has a solidification range above room temperature, by heating the components, with intimate homogeneous mixing, to a temperature above the miscibility gap, by rapidly cooling in a cooling liquid the solution thus obtained from the temperature above the miscibility gap and simultaneously shaping it to form a flat or capillary membrane and subsequently freeing the membrane from residual solvents and nonsolvents by extraction.

10. Method according to claim 9, characterised in that a 20 to 35% by weight solution is prepared.

11. Method according to one or both of claims 9 and 10, characterised in that a solution having a temperature of from 160 to 230°C is prepared.

12. Method according to one or more of claims 9 to 11, characterised in that a solution is prepared which contains from 80 to 95% by weight of polyvinylidene fluoride and from 5 to 20% by weight of polymethyl acrylate or polyethyl acrylate, referred to the total weight of polymer.

13. Method according to one or more of claims 9 to 12, characterised in that a polyvinylidene fluoride having an average molecular weight of from 30,000 to 500,000 and a polymer formed essentially from polymethyl acrylate or polyethyl acrylate and having an average molecular weight of from 5,000 to 1,000,000 are used for the preparation of the solution.

14. Method according to claim 13, characterised in that a polyvinylidene fluoride having an average molecular weight of from 50,000 to 500,000 and a polymer formed essentially from polymethyl acrylate or polyethyl acrylate and having an average molecular weight of from 50,000 to 200,000 are used.

15. Method according to one or more of claims 9 to 14, characterised in that one or more compounds from the group including glyceryl triacetate, glyceryl diacetate, 2-(2-butoxyethoxy)ethyl acetate and ε-caprolactam is or are used as solvent in the preparation of the solution.

16. Method according to claim 15, characterised in that the solvent used is glyceryl triacetate or a mixture of glyceryl triacetate and ε-caprolactam.

17. Method according to one or more of claims 9 to 16, characterised in that the nonsolvent used is di-n-octyl adipate or castor oil or a mixture thereof.

18. Method according to one or more of claims 9 to 17, characterised in that the cooling liquid used is water, optionally with the addition of a surfactant.

19. Method according to one or more of claims 9 to 18, characterised in that isopropanol is used for the extraction of the membrane.

20. Method for the preparation of the flat or capillary membrane according to one or more of claims 1 to 6, characterised in that a 10 to 40% by weight solution, referred to the total weight thereof, is prepared from 70 to 98% by weight of polyvinylidene fluoride and from 2 to 30% by weight of a polymer formed essentially from polymethyl acrylate or polyethyl acrylate, referred to the total weight of polymer, using one or more aprotic solvents, the solution is formed into a flat or capillary membrane, the latter optionally with the aid of an internal liquid, and transformed into the solid phase by coagulation in a nonsolvent bath and the membrane is subsequently freed from residual solvent by extraction.

21. Method according to claim 20, characterised in that one or more compounds from the group including N-methylpyrrolidone, dimethyl sulphoxide, dioxane, dimethylformamide and dimethylacetamide is or are used as aprotic solvent in the preparation of the solution.

22. Method according to one or both of claims 20 and 21, characterised in that the temperature of the solution and the temperature of the nonsolvent bath is from 0 to 80°C.

23. Method according to claim 22, characterised in that the temperature of the solution and the temperature of the nonsolvent bath is from 20 to 50°C.

24. Method according to one or more of claims 20 to 23, characterised in that the nonsolvent used for the coagulation of the membrane is an alcohol possessing 1 to 12 C atoms, or water, or mixtures thereof.

25. Method according to one or more of claims 20 to 24, characterised in that an alcohol possessing 1 to 3 C atoms is used for the extraction of the membrane.

26. Method according to one or more of claims 20 to 25, characterised in that a polyvinylidene fluoride having an average molecular weight of from 30,000 to 500,000 and a polymer formed essentially from polymethyl acrylate or polyethyl acrylate and having an average molecular weight of from 5,000 to 1,000,000 are used for the preparation of the solution.

27. Method according to claim 26, characterised in that a polyvinylidene fluoride having an average molecular weight of from 50,000 to 500,000 and a polymer formed essentially from polymethyl acrylate or polyethyl acrylate and having an average molecular weight of from 50,000 to 200,000 are used.

28. Method for the preparation of the flat or capillary membrane according to one or both of claims 7 and 8, characterised in that the ester groups on the total surface of the membrane according to one or more of claims 1 to 6 are subjected to an at least partial hydrolysis and/or to an at least partial trans-esterification with an at least trivalent alcohol possessing 3 to 12 C atoms and/or to an at least partial aminolysis with an amino compound possessing 2 to 8 C atoms.

29. Method according to claim 28, characterised in that the ester groups on the total surface of the membrane are subjected for 1 to 20 hours at a temperature of from 40 to 80°C to an at least partial hydrolysis using concentrated sulphuric acid.

30. Method according to claim 28, characterised in that the ester groups on the total surface of the membrane are subjected for 1 to 20 hours, at a temperature of from 80 to 140°C and at a pH value of from 9 to 11, to an at least partial hydrolysis using an aqueous borax solution which is saturated at room temperature and may contain up to 5% by weight of lye.

31. Method according to claim 28, characterised in that the ester groups on the total surface of the membrane are subjected for 1 to 30 hours, at a temperature of from 100 to 150°C to an at least partial trans-esterification using an at least trivalent alcohol possessing 3 to 12 C atoms with the addition of from 0.1 to 10% by weight, referred to the multivalent alcohol, of a strong acid.

32. Method according to claim 31, characterised in that one or more compounds from the group including glycerol, diglycerol, triglycerol, a polyglycerol mixture, pentaerythritol and sorbitol is or are used as the at least trivalent alcohol.

33. Method according to claim 28, characterised in that the ester groups on the total surface of the membrane are subjected for 1 to 24 hours, at a temperature of from 50 to 150°C and at a pH value of less than 11, to an at least partial aminolysis using at least one amino compound possessing 2 to 8 C atoms, using appropriate buffer mixtures.

34. Method according to claim 33, characterised in that one or more compounds from the group including diethylenetriamine, triethylenetetramine and tetraethylenepentamine is or are used as the amino compound.

35. Use of the flat or capillary membrane according to one or both of claims 7 and 8 for the immobilisation of biochemically active compounds.

## Revendications

1. Membrane plane ou capillaire à base d'un mélange homogène de poly(fluorure de vinylidène) et d'un deuxième polymère que l'on peut rendre hydrophile par réaction chimique, caractérisée en ce qu'elle est composée d'un mélange homogène de 70 à 98 % en poids de poly(fluorure de vinylidène) et de 2 à 30 % en poids d'un polymère constitué essentiellement de poly(acrylate de méthyle et/ou d'éthyle) et en ce que la taille maximale des pores est située dans le domaine compris entre 0,005 et 10 »m

2. Membrane plane ou capillaire conforme à la revendication 1, caractérisée en ce qu'elle présente une taille de pores maximale située dans le domaine compris entre 0,01 et 2 »m.

3. Membrane plane ou capillaire conforme à la revendication 2, caractérisée en ce qu'elle présente une taille de pores maximale située dans le domaine compris entre 0,05 et 0,8 »m.

4. Membrane plane ou capillaire conforme à une ou plusieurs des revendications 1 à 3, caractérisée en ce que le poly(fluorure de vinylidène) a une masse moléculaire moyenne (en poids) comprise entre 30 000 et 500 000 et le polymère constitué essentiellement de poly(acrylate de méthyle et/ou d'éthyle) a une masse moléculaire moyenne comprise entre 5 000 et 1 000 000.

5. Membrane plane ou capillaire conforme à la revendication 4, caractérisée en ce que le poly(fluorure de vinylidène) a une masse moléculaire moyenne comprise entre 50 000 et 500 000 et le polymère constitué essentiellement de poly(acrylate de méthyle et/ou d'éthyle) a une masse moléculaire moyenne comprise entre 50 000 et 200 000.

6. Membrane plane ou capillaire conforme à une ou plusieurs des revendications 1 à 5, caractérisée en ce qu'elle est composée d'un mélange homogène de 80 à 95 % en poids de poly(fluorure de vinylidène) et de 5 à 20 % en poids d'un polymère constitué essentiellement de poly(acrylate de méthyle et/ou d'éthyle).

7. Membrane plane ou capillaire conforme à une ou plusieurs des revendications 1 à 6, caractérisée en ce qu'elle porte sur toute sa surface entre 0,001 et 10 mmol/g de membrane de groupes -OH, -NH₂ ou -COOH ou des mélanges de ces groupes fonctionnels hydrophiles.

8. Membrane plane ou capillaire conforme à la revendication 7, caractérisée en ce qu'elle porte sur toute sa surface entre 0,01 et 5 mmol/g de membrane de groupes-OH, -NH₂ ou -COOH ou des mélanges de ces groupes fonctionnels hydrophiles.

9. Procédé pour la préparation de la membrane plane ou capillaire conforme à une ou plusieurs des revendications 1 à 6, caractérisé en ce que l'on prépare une solution ayant une concentration de 10 à 40 % en poids, par rapport à son poids total, à partir de 70 à 98 % en poids de poly(fluorure de vinylidène) et de 2 à 30 % en poids d'un polymère constitué essentiellement de poly(acrylate de méthyle et/ou d'éthyle), par rapport au poids total des composants polymères, en utilisant un ou plusieurs solvants et un ou plusieurs non-solvants, laquelle solution présente à l'état liquide au dessus de la température ambiante un domaine de miscibilité totale et une lacune de miscibilité, ainsi qu'une température de solidification supérieure à la température ambiante, le procédé consistant à chauffer les composants sous forte agitation-homogénéisation jusqu'à une température supérieure à celle de la lacune de miscibilité, à refroidir rapidement dans un liquide de refroidissement la solution ainsi obtenue qui est à une température supérieure à la lacune de miscibilité, à former simultanément une membrane plane ou capillaire, puis à débarrasser par extraction la membrane de solvants et de non solvants résiduels.

10. Procédé conforme à la revendication 9, caractérisé en ce que l'on prépare une solution de 20 à 35 % en poids.

11. Procédé conforme à une ou deux des revendications 9 à 10, caractérisé en ce que l'on prépare une solution à une température comprise entre 160 et 230 °.

12. Procédé conforme à une ou plusieurs des revendications 9 à 11, caractérisé en ce que l'on prépare une solution qui contient, par rapport au poids total des composants polymères, de 80 à 95 % en poids de poly(fluorure de vinylidène) et de 5 à 20 % en poids de poly(acrylate de méthyle et/ou d'éthyle).

13. Procédé conforme à une ou plusieurs des revendications 9 à 12, caractérisé en ce que l'on utilise pour la préparation de la solution un poly(fluorure de vinylidène) ayant une masse moléculaire moyenne comprise entre 30 000 et 500 000 et polymère constitué essentiellement de poly(acrylate de méthyle et/ou d'éthyle) ayant une masse moléculaire moyenne comprise entre 50 000 et 1 000 000.

14. Procédé conforme à la revendication 13, caractérisé en ce que l'on utilise pour la préparation de la solution un poly(fluorure de vinylidène) ayant une masse moléculaire moyenne comprise entre 50 000 et 500 000 et un polymère constitué essentiellement de poly(acrylate de méthyle et/ou d'éthyle) ayant une masse moléculaire moyenne comprise entre 50 000 et 200 000.

15. Procédé conforme à une ou plusieurs des revendications 9 à 14, caractérisé en ce que l'on utilise comme solvant pour la préparation de la solution un ou plusieurs des composés du groupe formé par le triacétate de glycérol, le diacétate de glycérol, l'acétate de 2-(2-butoxyéthoxyéthyle) et l'ε-caprolactame.

16. Procédé conforme à la revendication 15, caractérisé en ce que l'on utilise comme solvant du triacétate de glycérol ou un mélange de triacétate de glycérol et d'ε-caprolactame.

17. Procédé conforme à une ou plusieurs des revendications 9 à 16, caractérisé en ce que l'on utilise, comme non-solvant, de l'adipate de di-n-octyle ou de l'huile de ricin ou un mélange de ces deux.

18. Procédé conforme à une ou plusieurs des revendications 9 à 17, caractérisé en ce que l'on utilise, comme liquide de refroidissement, de l'eau ajoutée d'un agent tensioactif.

19. Procédé conforme à une ou plusieurs des revendications 9 à 18, caractérisé en ce que l'on utilise pour l'extraction de la membrane de l'isopropanol.

20. Procédé pour la préparation d'une membrane plane ou capillaire conforme à une ou plusieurs des revendications 1 à 6, caractérisé en ce que l'on prépare une solution ayant une concentration de 10 à 40 % en poids, par rapport à son poids total, à partir de 70 à 98 % en poids de poly(fluorure de vinylidène) et de 2 à 30 % en poids d'un polymère constitué essentiellement de poly(acrylate de méthyle et/ou d'éthyle), par rapport au poids total des composants polymères, dans un ou plusieurs solvants aprotiques, en ce que l'on met cette solution sous forme d'une membrane plane ou capillaire, pour cette dernière éventuellement à l'aide d'un liquide interne, que l'on solidifie par coagulation dans un bain de non-solvant, et en ce que l'on soumet enfin la membrane à une extraction, la débarrassant ainsi de solvant résiduel.

21. Procédé conforme à la revendication 20, caractérisé en ce que l'on utilise comme solvant aprotique pour la préparation de la solution un ou plusieurs composés du groupe formé par la N-méthylpyrrolidone, le diméthylsulfoxyde, le dioxane, le diméthylformamide et le diméthylacétamide.

22. Procédé conforme à une ou deux des revendications 20 à 21, caractérisé en ce que la température de la solution et la température du bain de non-solvant est comprise entre 0 et 80 °C.

23. Procédé conforme à la revendication 22, caractérisé en ce que la température de la solution et la température du bain de non-solvant est comprise entre 20 et 50 °C.

24. Procédé conforme à une ou plusieurs des revendications 20 à 23, caractérisé en ce que l'on utilise, comme non-solvant pour la coagulation de la membrane, un alcool comportant de 1 à 12 atomes de carbone ou de l'eau ou des mélanges de ces deux.

25. Procédé conforme à une ou plusieurs des revendications 20 à 24, caractérisé en ce que l'on utilise pour l'extraction de la membrane un alcool comportant de 1 à 3 atomes de carbone.

26. Procédé conforme à une ou plusieurs des revendications 20 à 25, caractérisé en ce que l'on utilise pour la préparation de la solution un poly(fluorure de vinylidène) ayant une masse moléculaire moyenne comprise entre 30 000 et 500 000 et un polymère constitué essentiellement de poly(acrylate de méthyle et/ou d'éthyle) ayant une masse moléculaire moyenne comprise entre 5 000 et 1 000 000.

27. Procédé conforme à la revendication 26, caractérisé en ce que l'on utilise un poly(fluorure de vinylidène) ayant une masse moléculaire moyenne comprise entre 50 000 et 500 000 et un polymère constitué essentiellement de poly(acrylate de méthyle et/ou d'éthyle) ayant une masse moléculaire moyenne comprise entre 50 000 et 200 000.

28. Procédé de préparation d'une membrane plane ou capillaire conforme à une ou deux des revendications 7 à 8, caractérisé en ce que l'on soumet les fonctions ester de la surface totale de la membrane, conforme à une ou plusieurs des revendications 1 à 6, à une hydrolyse au moins partielle et/ou une transestérification au moins partielle par un alcool au moins trivalent comportant de 3 - 12 atomes de carbone et/ou une aminolyse au moins partielle par un composé aminé comportant de 2 - 8 atomes de carbone.

29. Procédé conforme à la revendication 28, caractérisé en ce que l'on hydrolyse au moins partiellement les groupes esters de la surface totale de la membrane par action de l'acide sulfurique concentré à une température comprise entre 40 et 80 °C et pendant 1 à 20 heures.

30. Procédé conforme à la revendication 28, caractérisé en ce que l'on hydrolyse au moins partiellement les groupes esters de la surface totale de la membrane par une solution aqueuse de borax saturée à température ambiante, pouvant contenir jusqu'à 5 % en poids d'un hydroxyde de métal alcalin, à une température comprise entre 80 et 140 °C et à un pH compris entre 9 et 11, pendant 1 à 20 heures.

31. Procédé conforme à la revendication 28, caractérisé en ce que l'on soumet les groupes esters de la surface totale de la membrane à une transestérification au moins partielle par un alcool au moins trivalent comportant de 3 à 12 atomes de carbone ajouté de 0,1 à 10 % en poids, par rapport à l'alcool polyvalent, d'un acide fort à une température comprise entre 100 et 150 °C, pendant 1 à 30 heures.

32. Procédé conforme à la revendication 31, caractérisé en ce que l'on utilise comme alcool au moins trivalent un ou plusieurs des composés du groupe formé par le glycérol, le diglycérol, le triglycérol, un mélange de polyglycérol, le pentaérythrol et le sorbitol.

33. Procédé conforme à la revendication 28, caractérisé en ce que l'on soumet les groupes esters de la surface totale de la membrane à une aminolyse au moins partielle par un composé aminé comportant de 2 à 8 atomes de carbone en utilisant des tampons correspondants à un pH < 11 et à une température comprise entre 50 et 150 °C, pendant 1 à 24 heures.

34. Procédé conforme à la revendication 33, caractérisé en ce que l'on utilise, comme composé aminé, un ou plusieurs des composés du groupe formé par la diéthylènetriamine, la triéthylènetétra-amine et la tétraéthylènepenta-amine.

35. Utilisation de la membrane plane ou capillaire conforme à une ou deux des revendications 7 à 8 pour l'immobilisation de composés à activité biologique.
